# FASCICULE DE BREVET EUROPEEN

(11) **EP 2 639 294 B1**
(45) Date de publication et mention de la délivrance du brevet: **24.06.2026**
(21) Numéro de dépôt: 12305310.0
(22) Date de dépôt: 15.03.2012
(51) Int. Cl.: C12M 3/00, C12M 1/00

(54) **Automate et procédé automatisé de culture cellulaire**
Automat und automatisiertes Verfahren zur Herstellung von Zellkulturen
Automaton and automated method for cell culture

(43) Date de publication de la demande: 18.09.2013
(73) Titulaire: CellProthera, 68100 Mulhouse (FR)
(72) Inventeur: Henon, Philippe, 68100 Mulhause (FR); Saucourt, Claire, 68100 Mulhause (FR); Gasse, Patrick, 78310 Maurepas (FR); Sundas, Alain, 78330 Fontenay-le-Fleury (FR); Sugranes, Pierre, 78700 Conflans-Sainte-Honorine (FR); Verdier, Amandine, 92240 Malakoff (FR); Demonchy, Frederic, 78990 Elancourt (FR)
(74) Mandataire: Ernest Gutmann - Yves Plasseraud S.A.S.

(56) Documents cités:
- EP-A1- 1 944 359
- EP-A1- 1 978 089
- EP-A2- 1 932 904
- EP-A2- 2 325 297
- WO-A2-2011/005773
- DE-A1- 102006 022 652
- US-A- 4 829 002
- US-A- 5 352 213
- US-A1- 2003 064 503
- US-A1- 2004 110 274

## Description

La présente invention se rapporte à un automate de culture cellulaire, en particulier pour la culture de cellules souches (du type CD34+ par exemple), ainsi qu'à un procédé de culture cellulaire utilisant cet automate.

Parmi les domaines faisant appel à la culture cellulaire, celui de la thérapie cellulaire est le moins avancé en termes d'industrialisation. Il existe donc un besoin important de trouver une technologie capable de produire des cellules en quantité suffisante et dans des conditions optimales en vue de l'utilisation de ces cellules pour des applications thérapeutiques.

Certains procédés de thérapie cellulaire nécessitent une culture ou amplification de cellules souches avant leur réinjection chez un patient, car les quantités prélevées sont parfois insuffisantes pour avoir un effet thérapeutique. Il est indispensable de garantir l'intégrité des propriétés thérapeutiques des cellules pendant leur culture. Dans la technique actuelle, les solutions proposées pour cultiver les cellules souches ex *vivo* sont artisanales, très empiriques et peu efficaces.

De plus, la technique actuelle ne permet pas de produire des cellules souches en quantité suffisante pour des applications thérapeutiques. Il existe donc un réel besoin de développer une technologie du type bioréacteur ayant une géométrie compacte et permettant de cultiver des cellules en grande quantité.

On a déjà proposé d'utiliser des bioréacteurs pour la culture de cellules souches. Cependant, la phase d'amplification reste une étape essentiellement manuelle et les conditions environnementales pour la culture des cellules (température, CO₂, etc.) ne sont pas contrôlées avec une grande précision.

On connaît par exemple des bioréacteurs avec perfusion à membrane, des bioréacteurs à fibres creuses, des bioréacteurs sur lit fluidisé, et des micro-bioréacteurs avec perfusion continue d'O2, de milieu nutritif et de facteurs de croissance (cf. demande WO 00/73411).

La demande US-A1-2008/0118977 décrit un protocole thérapeutique permettant de reconstruire le cœur d'un patient après un infarctus. La reconstruction est obtenue en injectant au niveau du cœur du patient des cellules souches spécifiques (CD34+), isolées à partir d'un prélèvement sanguin, amplifiées ex vivo et purifiées après culture.

Il est connu le document EP 1 978 089 A1.

La présente invention a notamment pour but de fournir une technologie capable d'assurer la culture, l'amplification ou l'expansion de ce type de cellules, et d'apporter également de nettes améliorations en termes de standardisation, de traçabilité et de contrôle pour des opérations qui sont pour la plupart réalisées manuellement dans la technique antérieure.

Elle propose à cet effet un automate de culture cellulaire, comprenant des réservoirs de milieu de culture, de facteurs de croissance et de cellules à cultiver, un incubateur à enceinte thermostatée dans laquelle est logé un récipient de culture des cellules ou d'expansion cellulaire, et un système informatique de commande incluant des moyens de saisie et d'enregistrement de données et destiné à réguler les conditions de culture dans l'enceinte et à piloter des vannes de distribution de fluides selon une séquence prédéfinie, caractérisé en ce qu'il comprend un dispositif de support et d'agitation du récipient de culture ou d'expansion cellulaire qui est commandé par le système informatique et qui est logé dans l'enceinte, et en ce que le récipient est formé par une poche d'expansion cellulaire comportant au moins un orifice d'entrée relié aux réservoirs précités et un orifice de sortie relié à des moyens de récupération et de stockage des cellules après culture, ces moyens de stockage et les réservoirs étant situés à l'extérieur de l'enceinte et étant reliés aux orifices de la poche d'expansion cellulaire par des conduits qui forment avec la poche d'expansion cellulaire un module pré-assemblé posé dans l'enceinte et qui traversent un élément de paroi de l'enceinte, de façon à permettre d'alimenter la poche d'expansion cellulaire en milieu de culture, en facteurs de croissance et en cellules à cultiver, et de récupérer le contenu de la poche d'expansion cellulaire dans les moyens de stockage, en maintenant l'enceinte fermée. Le dispositif de support et d'agitation comprend un plateau de support de la poche d'expansion cellulaire, qui est monté rotatif autour d'un premier axe horizontal et qui est déplaçable autour de cet axe entre une position sensiblement horizontale de culture des cellules et une position sensiblement verticale de récupération des cellules après culture.

L'automate selon l'invention a essentiellement pour fonction d'automatiser les étapes du protocole biologique de culture et la gestion des conditions environnementales (contrôle de la température, du taux de CO2, etc.) de culture des cellules de l'incubateur, afin de produire un rendement optimal d'amplification cellulaire. Il a également pour fonction d'assurer la distribution du milieu de culture, des facteurs de croissance et des cellules à cultiver à une poche d'expansion cellulaire (qui peut avoir un volume relativement important) située dans l'incubateur, par la commande de moyens tels que des vannes et des pompes. L'automate assure en outre l'agitation de la poche d'expansion cellulaire ainsi que le transfert des cellules après culture, depuis la poche d'expansion cellulaire jusqu'aux moyens de stockage.

L'automate selon l'invention peut permettre de générer de grandes quantités de cellules, telles que des cellules souches, à partir de cellules prélevées sur un patient. La poche dans laquelle est réalisée la culture cellulaire peut avoir un volume supérieur à 100ml, 200ml, 300ml, 500 ml, et qui est par exemple de 650 ml environ voire plus (1L, 2L, 3L, etc.). La culture de cellules souches dans une poche de ce type permet de générer des cellules en quantité suffisante pour réaliser une thérapie cellulaire chez un patient, tel par exemple qu'un patient ayant subi un infarctus, selon le protocole biologique décrit dans la demande US-A1-2008/0118977.

La poche d'expansion cellulaire comprend de préférence des parois souples étanches aux liquides et perméables aux gaz. Elle présente de préférence une bonne perméabilité à l'oxygène et au dioxyde de carbone, ce qui permet une bonne aération du contenu de la poche sans ouverture de celle-ci et donc sans risque de contamination de son contenu. Dans un exemple particulier de réalisation de la poche, elle comprend les caractéristiques de perméabilité suivantes (en cc par jour, à 37°C) : O₂ (gaz) ≈ 418, CO₂ (gaz) ≈ 966, N₂ (gaz) ≈ 157, et H₂O (liquide) ≈ 0,05.

La poche d'expansion cellulaire a de préférence peu d'affinité avec des produits chimiques et biologiques, en particulier avec les cellules à cultiver, et n'absorbent pas de tels produits. La poche est par exemple formée d'un film mince en copolymère FEP (fluoro-éthylène-propylène). La poche peut être constituée de différents types de ports (interfaces modifiables) dont, par exemple, des raccords sapin en FEP. Ils sont assemblés à la poche pour limiter les risques de contamination.

L'automate est destiné à réaliser la culture cellulaire et inclut tous les moyens et toutes les ressources pour réaliser cette culture, sans nécessité pour un opérateur de manipuler de vannes, remplacer des poches ou des réservoirs, etc. La culture cellulaire est réalisée selon un protocole biologique précis qui est entièrement géré par le système informatique, qui permet par exemple de commander les vannes, le dispositif d'agitation, et de réguler les conditions environnementales dans l'enceinte de l'incubateur. L'opérateur peut renseigner le système informatique avec des données d'identification du patient, des cellules prélevées et la nature et la provenance des différents réservoirs ou poches, afin que toutes ces données soient enregistrées dans le système informatique. L'invention permet ainsi de réaliser des protocoles biologiques avec une très bonne reproductibilité et d'assurer une traçabilité et un contrôle précis des protocoles et moyens utilisés.

Le contrôle et la traçabilité des étapes du protocole biologique peuvent être assurés par le système informatique et une interface homme-machine (IHM) appropriée, qui permettent par exemple de :
- définir un procédé de culture automatisé dans lequel les paramètres propres au protocole biologique de culture ne sont pas modifiables,
- assurer une bonne sécurité en limitant l'accès aux données du système informatique, par l'identification de l'utilisateur et la nécessité d'un mot de passe (conformément à la réglementation 21 CFR Part 11 de la FDA),
- autoriser un enregistrement des événements et des différentes étapes du procédé, et
- établir une édition de rapports (comprenant des résultats d'analyse de prélèvement et d'analyse a posteriori des caractéristiques du greffon par exemple).

La poche d'expansion cellulaire est logée dans l'enceinte et comprend au moins deux orifices qui sont reliées à des conduits qui passent à travers un élément de paroi de l'incubateur, et qui sont reliés aux réservoirs et aux moyens de stockage reliés à l'extérieur de l'enceinte. La poche d'expansion cellulaire et les conduits forment un module pré-assemblé, à usage unique, qui est mis en place et remplacé facilement par l'opérateur. Au moins une partie du contenu des réservoirs situés à l'extérieur de l'enceinte est destinée à être distribuée dans la poche d'expansion cellulaire située dans l'enceinte, dont le contenu après culture est destiné à être transféré dans les moyens de stockage situés hors de l'enceinte, toutes ces opérations de distribution de fluides étant réalisées en maintenant l'enceinte de l'incubateur fermée grâce aux conduits qui traversent un élément de paroi de l'incubateur, ce qui permet de garantir dans l'enceinte des conditions environnementales optimales pendant toute la durée du protocole biologique et de limiter les risques de contamination du milieu de culture des cellules.

Avantageusement, la poche d'expansion cellulaire comprend un orifice de prélèvement qui est relié par un conduit à des moyens de prélèvement situés à l'extérieur de l'enceinte, ce conduit traversant l'élément de paroi précité de l'enceinte et faisant partie du module pré-assemblé. La poche d'expansion cellulaire comprend ainsi trois orifices ayant des fonctions différentes (alimentation récupération et prélèvement) et qui sont reliés à des conduits différents.

Dans un mode de réalisation de l'invention, l'incubateur comprend une armoire comportant une ouverture et une porte de fermeture étanche de cette ouverture, des moyens de passage des conduits précités étant montés sur le bord périphérique de cette ouverture et comportant des gorges sensiblement parallèles de logement des conduits, ces gorges étant destinées à être recouvertes par la porte lorsqu'elle est en position fermée. Les conduits peuvent être aisément engagés (et retirés) de ces conduits par un opérateur quand la porte est ouverte, par translation des conduits dans une direction perpendiculaire aux axes longitudinaux des gorges, ce qui facilite le montage des consommables.

Les réservoirs de facteurs de croissance et de cellules à cultiver sont de préférence formés par des poches qui sont situées plus haut que l'orifice d'entrée de la poche d'expansion cellulaire de façon à ce que le contenu de chacune des poches de milieu de culture et de cellules à cultiver puisse s'écouler par gravité jusqu'à la poche d'expansion cellulaire. Ceci permet de garantir l'intégrité des cellules et des facteurs de croissance lors de leur transfert jusqu'à la poche d'expansion cellulaire. L'utilisation d'une pompe ou d'un moyen mécanique quelconque pour faire circuler les cellules et les facteurs de croissance dans les conduits risquerait en effet de les endommager.

Les moyens de stockage peuvent comprendre une ou deux poches qui sont au moins en partie situées plus bas que l'orifice de sortie de la poche d'expansion cellulaire de façon à ce que, après culture, le contenu de la poche d'expansion cellulaire puisse s'écouler par gravité jusqu'à la ou les poches des moyens de stockage. Ceci permet également de garantir l'intégrité des cellules après culture, lors de leur récupération

L'automate peut comprendre une pompe péristaltique de commande de l'alimentation en milieu de culture de la poche d'expansion cellulaire et des réservoirs de facteurs de croissance et de cellules à cultiver, en vue du rinçage de ces réservoirs. La pompe péristaltique a l'avantage de ne pas venir directement au contact du milieu de culture, ce qui évite tout risque de contamination de ce milieu.

L'automate peut en outre comprendre deux poches formant piège à air, dont l'une est reliée aux réservoirs de facteurs de croissance et de cellules à cultiver, et dont l'autre est reliée à la poche d'expansion cellulaire, et qui sont destinées à collecter et stocker l'air contenu dans les conduits, la poche d'expansion cellulaire et/ou les réservoirs.

Avantageusement, les conduits sont formés par des tubulures souples, dont au moins certaines traversent des vannes qui sont destinées, en position de fermeture, à pincer ces tubulures. Chaque tubulure est par exemple destinée à être engagée dans une gorge d'une vanne de façon simple par l'opérateur, par exemple par translation dans une direction sensiblement perpendiculaire à l'axe longitudinal de la tubulure.

Comme décrit précédemment, le dispositif de support et d'agitation comprend un plateau de support de la poche d'expansion cellulaire, qui est monté rotatif autour d'un premier axe horizontal et qui est déplaçable autour de cet axe entre une position sensiblement horizontale de culture des cellules et une position sensiblement verticale de récupération des cellules après culture. Cette dernière position facilite la récupération des cellules après culture, ces cellules s'écoulant directement par gravité dans les moyens de stockage précités.

Le plateau peut être monté rotatif autour d'un second axe horizontal autour duquel le plateau est destiné à osciller pour l'agitation et l'homogénéisation du contenu de la poche d'expansion cellulaire. Les premier et second axes de rotation du plateau sont de préférence parallèles.

De préférence, le plateau porte des vannes de commande de l'alimentation de la poche d'expansion cellulaire, de récupération du contenu de cette poche, et de prélèvement d'échantillons de cette poche.

Le dispositif de support et d'agitation peut en outre comprendre un bras vertical comportant à son extrémité supérieure des moyens d'accrochage d'une poche formant piège à air reliée à la poche d'expansion cellulaire.

Les moyens de stockage sont avantageusement montés rotatifs autour d'un axe horizontal et sont déplaçables autour de cet axe entre une position sensiblement verticale et une position sensiblement horizontale dans laquelle ces moyens sont entièrement situés en dessous de la poche d'expansion cellulaire. Ceci permet de garantir que l'intégralité des cellules contenues dans la poche d'expansion cellulaire sera transférée par gravité dans les moyens de stockage.

A titre d'exemple, dans le cas particulier de culture de cellules souches CD34+, la stérilité exigée au niveau de la chaîne complète de conditionnement des cellules CD34+ impose l'utilisation et l'installation de consommables sous forme d'un kit de culture cellulaire à usage unique.

La présente invention concerne donc également un kit de culture cellulaire, de préférence stérile et à usage unique, pour un automate de culture cellulaire, caractérisé en ce qu'il comprend au moins une poche d'expansion cellulaire et des tubulures souples de liaison de la poche à d'autres poches ou réservoirs, les tubulures et la poche d'expansion cellulaire formant un module pré-assemblé, la poche étant destinée à être posée dans une enceinte de culture cellulaire et les tubulures étant destinées à traverser un élément de paroi de ladite enceinte, la poche d'expansion cellulaire comprenant un orifice d'entrée, un orifice de sortie et éventuellement un orifice de prélèvement.

Le kit peut en outre comprendre tous les raccords nécessaires aux liaisons des tubulures entre elles et avec les poches et/ou réservoirs, ainsi que les moyens reliés au troisième orifice de la poche d'expansion cellulaire pour autoriser des prélèvements de cellules. Tous ces éléments peuvent faire partie du module pré-assemblé précité.

L'orifice d'entrée de la poche d'expansion cellulaire est relié par des tubulures à des orifices d'entrée et de sortie de la poche de facteurs de croissance, et à des orifices d'entrée et de sortie de la poche de cellules à cultiver. L'orifice d'entrée de la poche d'expansion cellulaire est en outre destiné à être reliée à un orifice de sortie de la poche d'expansion cellulaire.

Le kit comprend en outre deux poches formant piège à air dont l'une est reliée aux orifices de sortie des poches de facteurs de croissance et de cellules à cultiver, et dont l'autre est reliée à l'orifice d'entrée de la poche d'expansion cellulaire.

Le kit peut également comprendre une ou deux poches de récupération des cellules après culture, qui sont reliées par des tubulures à l'orifice de sortie de la poche d'expansion cellulaire.

Dans un exemple de réalisation de l'invention, les poches de facteurs de croissance et de cellules à cultiver et celles formant piège à air ont un volume interne de 150 ml environ, la poche d'expansion cellulaire a un volume théorique de 3000mL environ, et les deux poches de récupération ont chacune un volume de 600 ml environ. La poche de distribution du milieu de culture peut avoir un volume de 1000 ml environ.

Avantageusement, le kit forme ou constitue un circuit fermé qui, une fois installé pour une culture cellulaire, comprend toutes les ressources nécessaires pour cette culture sans nécessiter l'ajout d'un produit quelconque ou l'intervention d'un opérateur. Ceci permet de limiter les risques de contamination du kit et du milieu de culture.

L'invention concerne encore un dispositif de support et d'agitation pour un automate de culture cellulaire, caractérisé en ce qu'il comprend un plateau de support d'une poche d'expansion cellulaire, ce plateau portant trois vannes et étant monté en rotation autour d'un premier axe horizontal pour le basculement du plateau depuis une position sensiblement horizontale jusqu'à une position sensiblement verticale, et un second axe horizontal autour duquel le plateau est destiné à osciller pour l'agitation et l'homogénéisation du contenu de la poche d'expansion cellulaire, le dispositif comprenant également des moyens commandés de basculement du plateau autour des axes horizontaux précités.

Le dispositif peut comprendre un bras vertical comportant à son extrémité supérieure des moyens d'accrochage d'une poche formant piège à air.

L'invention concerne également un procédé automatisé de culture cellulaire, au moyen d'un automate tel que décrit ci-dessus, caractérisé en ce qu'il comprend les étapes consistant à :
a) alimenter la poche d'expansion cellulaire en milieu de culture, en facteurs de croissance puis en cellules à cultiver, en maintenant l'enceinte de l'incubateur fermée ;
b) agiter la poche d'expansion cellulaire en vue de l'homogénéisation de son contenu ;
c) maintenir la poche d'expansion cellulaire dans des conditions d'incubation pendant une durée de plusieurs jours ; et
d) récupérer le contenu de la poche d'expansion cellulaire dans les moyens de stockage, en maintenant l'enceinte fermée.

Le procédé selon l'invention peut comprendre une ou plusieurs des étapes suivantes :
- avant l'étape a), une étape d'installation du module pré-assemblé en montant la poche d'expansion cellulaire sur le dispositif d'agitation, en montant les conduits dans les moyens de passage de l'incubateur et dans les vannes, et en connectant ces conduits aux réservoirs ou poches,
- avant l'étape a), une étape d'évacuation de l'air contenu dans les conduits par passage de milieu de culture depuis le réservoir de milieu de culture jusqu'à la ou les poches formant piège à air ;
- après l'alimentation de la poche d'expansion cellulaire en facteurs de croissance à l'étape a), une étape de rinçage du réservoir de facteurs de croissance en faisant passer du milieu de culture dans ce réservoir puis en évacuant son contenu jusqu'à la poche d'expansion cellulaire ;
- après l'alimentation de la poche d'expansion cellulaire en cellules à cultiver à l'étape a), une étape de rinçage du réservoir de cellules à cultiver en faisant passer du milieu de culture dans ce réservoir puis en évacuant son contenu jusqu'à la poche d'expansion cellulaire ;
- pendant l'étape c), une ou plusieurs étapes de prélèvement d'échantillon du contenu de la poche d'expansion cellulaire, qui sont chacune précédées d'une étape de basculement du plateau de support d'une position horizontale de culture jusqu'à une position inclinée dans laquelle l'orifice de prélèvement de la poche représente le point le plus bas de la poche ;
- avant l'étape c), une étape de retrait des réservoirs de milieu de culture, de facteurs de croissance et de cellules à cultiver, en coupant et en soudant ou pinçant le conduit ou la tubulure de liaison de ces réservoirs à l'orifice d'entrée de la poche d'expansion cellulaire ;
- avant ou pendant l'étape d), à basculer le plateau dans une position sensiblement verticale de façon à ce que l'orifice de sortie de la poche d'expansion cellulaire représente le point le plus bas de la poche.

L'invention concerne enfin une utilisation d'un automate, d'un kit, ou d'un dispositif tel que décrit ci-dessus, pour la culture de cellules souches du type CD34+ ou de cellules mononuclées du sang, telles que par exemple des lymphocytes. Les cellules souches peuvent provenir d'une ou plusieurs sources, comme plus particulièrement le sang de cordon ombilical, la moelle osseuse et le sang total.

L'invention sera mieux comprise et d'autres détails, avantages et caractéristiques de l'invention apparaîtront plus clairement à la lecture de la description suivante faite à titre d'exemple non limitatif et en référence aux dessins annexés dans lesquels :
- les figures 1 et 2 sont des vues schématiques en perspective de l'automate de culture cellulaire selon l'invention, cet automate comportant une armoire définissant une enceinte qui est fermée en figure 1 et ouverte en figure 2 ;
- la figure 3 est une vue très schématique de l'automate des figures 1 et 2, sans le système informatique ;
- la figure 4 est une vue très schématique des composants portés par un dispositif d'agitation de l'automate des figures 1 et 2 ;
- la figure 5 est une vue schématique d'un kit de culture cellulaire selon l'invention ;
- la figure 6 est une vue schématique en perspective de moyens de passage de conduits de fluide de l'automate des figures 1 et 2 ;
- la figure 7 est une vue schématique en perspective du dispositif d'agitation selon l'invention ;
- les figures 8 et 9 sont des vues schématiques en perspective du dispositif de la figure 7 et représentent deux positions d'inclinaison différentes du plateau de ce dispositif ;
- la figure 10 est une autre vue schématique en perspective du dispositif d'agitation de la figure 7, avec arrachement partiel du capotage de ce dispositif ;
- la figure 11 est une vue schématique en perspective du plateau et de moyens commandés de basculement du plateau du dispositif de la figure 7, vus de dessous ;
- les figures 12 et 13 sont vues schématiques en perspective d'un système commandé de blocage de rotation du plateau du dispositif de la figure 7, ce système de blocage étant actif en figure 11 et inactif en figure 12 ;
- la figure 14 est un organigramme montrant des étapes d'un procédé de culture cellulaire selon l'invention ; et
- les figures 15 à 24 sont des vues correspondant à la figure 3 et représentant des étapes du procédé selon l'invention.

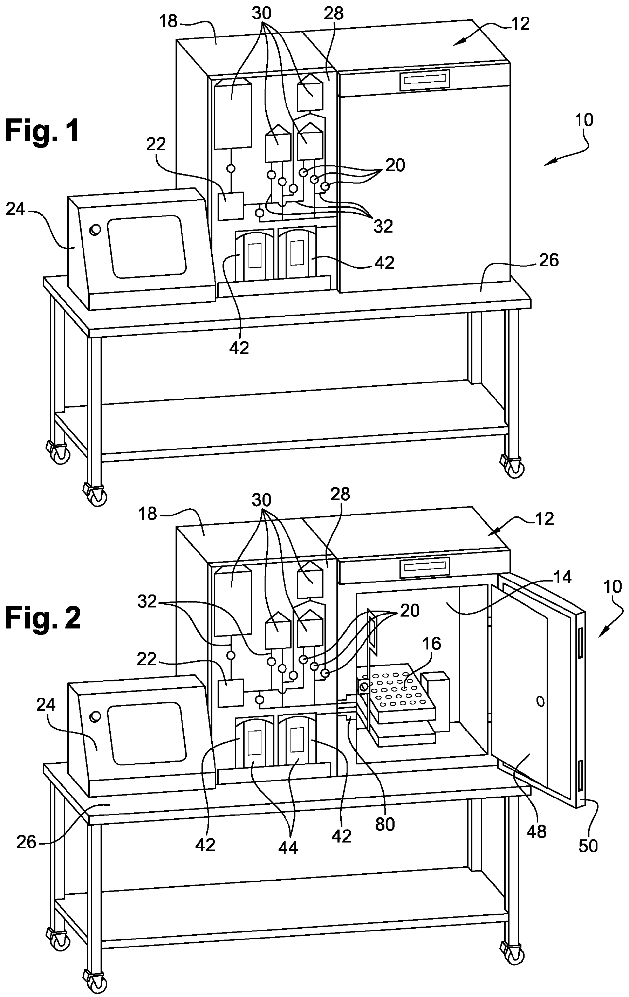

On se réfère d'abord aux figures 1 et 2 qui représentent un exemple de réalisation de l'automate 10 de culture cellulaire selon l'invention, cet automate étant particulièrement mais non exclusivement destiné à la culture de cellules souches, par exemple selon le protocole biologique décrit dans la demande US-A1-2008/0118977 dont le contenu est incorporé ici par référence.

Dans l'exemple représenté, l'automate 10 comprend essentiellement trois éléments :
- un incubateur 12 à enceinte 14 thermostatée dans laquelle est logé un dispositif 16 de support et d'agitation d'une poche d'expansion cellulaire (non représentée),
- un bâti 18 de support de poches (non représentées) contenant les milieux nécessaires à la culture des cellules, et portant des moyens (vannes 20, pompe 22, etc.) de distribution et de réglage de débit de fluides entre les poches, et
- un système informatique 24 relié à l'incubateur 12 et aux moyens 20, 22 pour leur commande ainsi que pour la saisie et l'enregistrement de données et la gestion du protocole biologique.

Dans l'exemple représenté, l'incubateur 12, le bâti 18 et le système informatique 24 sont disposés l'un à côté de l'autre sur un support 26 qui est monté sur roulettes, le bâti 18 étant situé entre le système informatique 24 et l'incubateur 12.

De façon typique, le système informatique 24 comprend des moyens de saisie et d'enregistrement de données, des moyens de traitement des données, des moyens d'affichage, et des moyens d'émission de signaux de commande et de pilotage de l'incubateur 12 et des moyens 20, 22 du bâti 18. De préférence, le système informatique 24 comprend un écran tactile d'affichage et de saisie de données.

Pour limiter l'accès aux données préenregistrées dans le système informatique 24, plusieurs niveaux de sécurité peuvent être mis en place. Le constructeur de l'automate peut avoir un droit d'accès de niveau maximal, au moyen d'un mot de passe spécifique, de façon à accéder à toutes les informations enregistrées dans le système informatique 24, alors qu'un administrateur et un opérateur ayant des niveaux d'accès inférieurs auront accès, via des mots de passe spécifiques, à certaines informations seulement.

Le système informatique 24 est avantageusement relié à un réseau informatique via une connexion du type Ethernet ou Wifi par exemple, de façon à ce que des informations contenues dans le système 24 puissent être accessibles depuis un poste informatique du réseau, distant de l'automate 10, et éventuellement que des actions puissent être requises et commandées à l'automate depuis ce poste.

Le système informatique 24 commande par exemple l'ouverture et la fermeture des vannes 20, qui sont par exemple du type tout ou rien, le réglage du débit de la pompe 22, la régulation du chauffage de l'enceinte 14 de l'incubateur 12 (pour qu'elle ait par exemple une température de l'ordre de 37°C environ) et l'alimentation de l'enceinte en gaz, tels que du CO₂ (à un taux d'environ 5% par exemple). Le système 24 peut assurer la régulation d'autres paramètres si nécessaire à l'intérieur de l'enceinte 12, pour définir des conditions environnementales optimales pour la culture des cellules.

Pour plus de clarté, les moyens de connexion du système informatique 24 aux moyens 18, 20 et à l'incubateur 12, les moyens de chauffage et d'alimentation en gaz de l'incubateur 12, et les moyens d'alimentation électrique ne sont pas représentés dans les dessins.

Le bâti 18 a une forme parallélépipédique et comprend une face verticale avant 28 sur laquelle sont schématiquement dessinés des rectangles 30 représentant les positions de réservoirs de milieux biologiques, sous forme de poches, ainsi que des lignes 32 représentant l'emplacement de conduits de fluide(s) entre ces poches.

La partie supérieure de cette face avant 28 comprend quatre rectangles 30 dessinés qui renseignent un opérateur sur la nature de chacune des poches qui doivent être positionnées au niveau de ces rectangles, ces poches appartenant à un kit de consommables qui sera décrit plus en détail dans ce qui suit.

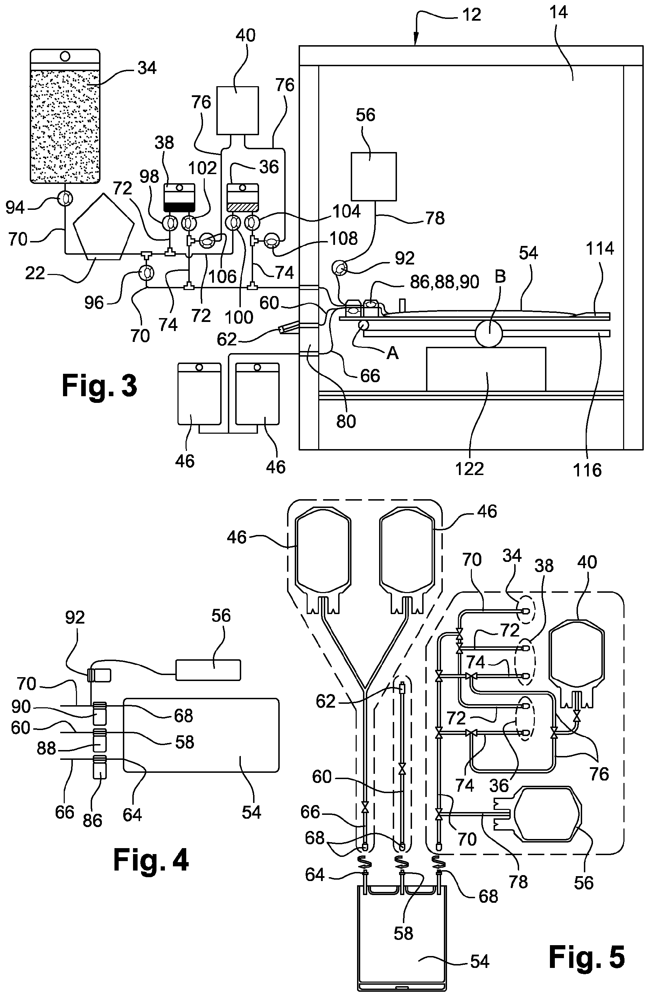

Un premier rectangle de grande dimension est dessiné en haut à gauche de la face avant 28 du bâti 18 et représente la position d'une poche contenant un milieu de culture (poche référencée 34 en figure 3). Trois rectangles 30 de plus petites dimensions sont dessinés en haut à droite de la face 28 et représentent respectivement les positions d'une poche de facteurs de croissance, d'une poche de cellules à cultiver, et d'une poche formant piège à air (qui sont respectivement référencées 36, 38 et 40 en figure 3).

La partie médiane de la face avant 28 du bâti 18 comprend des orifices de montage des vannes 20 et de la pompe 22 précitées, chacun de ces éléments (vannes et pompe) étant situé sur une ligne 32 représentant un conduit de fluide, qui est formé par une tubulure souple du kit de consommables.

La partie inférieure de la face avant 28 porte deux plaques 42 coplanaires et disposées l'une à côte de l'autre. Ces plaques 42 sont montées pivotantes par leurs extrémités inférieures autour d'un même axe horizontal s'étendant parallèlement à la face avant 28. Les plaques 42 sont déplaçables en rotation autour de cet axe entre une position verticale (représentée en figures 1 et 2) dans laquelle elles s'étendent parallèlement et à faible distance de la face avant 28, et une position horizontale dans laquelle elles peuvent prendre appui sur le support 26.

Des rectangles 44 sont dessinés sur les faces avant des plaques 42, lorsqu'elles sont en position verticale. Ces rectangles 44 renseignent l'opérateur sur la nature des poches qui doivent être portées par ces plaques 42. Des poches de récupération et de stockage des cellules après culture (référencées 46 en figure 3) sont destinées à être portées par ces plaques 42.

Les poches 34, 36, 38, 40 et 46 du kit de consommables sont destinées à être fixées ou accrochées sur la face avant 28 du bâti 18 et sur les plaques 42 par des moyens appropriés qui ne sont pas représentés.

L'incubateur 12 comprend une armoire définissant l'enceinte 14 et comportant une ouverture qui peut être fermée de manière étanche par une double porte, ces deux portes 48, 50 étant montées pivotantes sur un des côtés de l'ouverture, par exemple le côté droit.

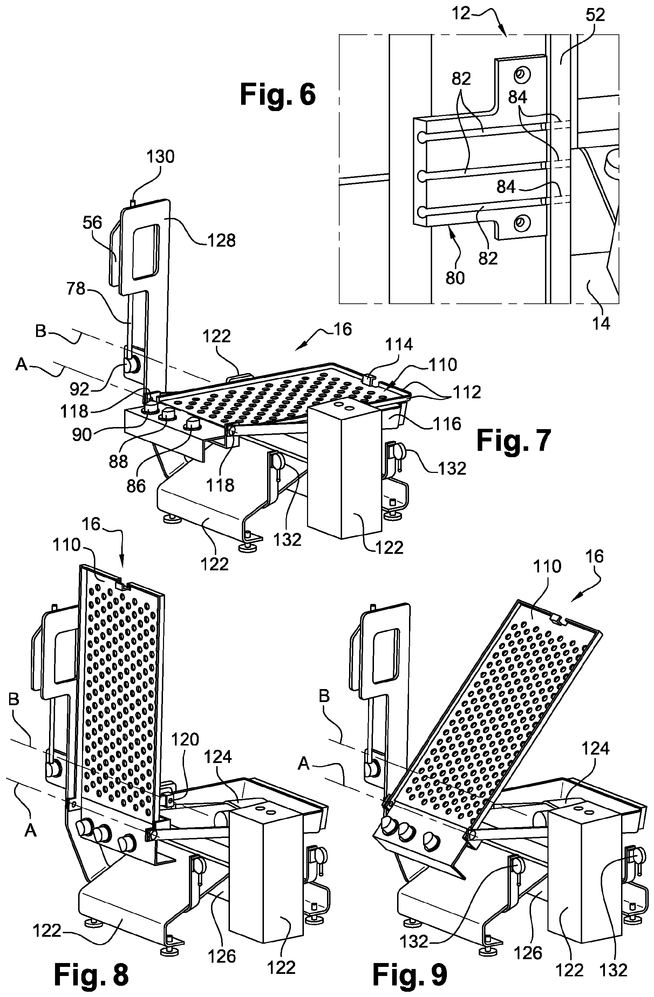

La porte interne 48 est une porte en verre destinée en position de fermeture à prendre appui sur un joint périphérique 52 de l'ouverture de l'armoire, ce joint 52 étant visible en figure 6. La porte externe 50 est calorifugée et porte un joint périphérique destinée à prendre appui sur le bord périphérique de l'ouverture de l'armoire.

Le système informatique 24 peut être relié à des capteurs de détection de la position (ouverte ou fermée) de chaque porte 48, 50, et peut commander le verrouillage de ces portes en particulier pendant la phase d'incubation et de culture des cellules.

L'enceinte 14 de l'incubateur 12 a par exemple un volume interne de 200 L environ.

Dans l'exemple représenté aux figures 3 à 5, le kit de consommables est à usage unique pour une culture cellulaire et comprend les poches 34, 36, 38, 40 et 46 et les tubulures précitées, ainsi qu'une poche d'expansion cellulaire 54 et une seconde poche 56 formant piège à air, ces poches 54, 56 étant portées par le dispositif d'agitation 16 qui sera décrit plus en détail dans ce qui suit, en référence aux figures 7 à 13.

La poche d'expansion cellulaire 54 mieux visible aux figures 4 et 5 peut avoir un volume interne supérieur à 500 ml, et qui est par exemple de 650 ml, et comprend trois orifices, un orifice de prélèvement 58 relié par une tubulure 60 à des moyens de prélèvement 62, un orifice de sortie 64 relié par une tubulure 66 aux poches 46 de récupération des cellules après culture, et un orifice d'entrée 68 reliée par des tubulures aux poches 34, 36, 38 et 56.

L'orifice d'entrée 68 de la poche d'expansion cellulaire 54 est relié par une tubulure 70 à un orifice d'entrée de la poche 34 de milieu de culture. Les poches 36 et 38 de facteurs de croissance et de cellules à cultiver comprennent chacune un orifice d'entrée qui est relié à une extrémité d'une tubulure 72 dont l'autre extrémité est raccordée à la tubulure 70, et un orifice de sortie qui est relié à une extrémité d'une tubulure 74 dont l'autre extrémité est raccordée à la tubulure 70 (en aval du ou des points de connexion des tubulures 72 à la tubulure 70). La poche 40 comprend deux orifices qui sont reliés par des tubulures 76 aux tubulures 74, et la poche 56 formant piège à air comprend un orifice relié par une tubulure 78 à la tubulure 70, au voisinage de l'orifice d'entrée 68 de la poche 54 (figures 4 et 5).

La poche d'expansion cellulaire 54 et les tubulures 60, 66, 70, 72, 74, 76 et 78 sont de préférence pré-assemblées et sont fournies stériles. Les poches 34, 36, 38, 40, 46 et 56 sont également fournies stériles. Les poches 40, 46 et 56 sont fournies vides et peuvent être pré-assemblées avec la poche d'expansion cellulaire 54 aux tubulures précitées. La poche 38 de cellules à cultiver est également fournie vide et peut être pré-assemblée aux tubulures ou bien connectée aux tubulures lors de l'installation du kit dans l'automate. La poche 38 peut être remplie d'un milieu contenant les cellules à cultiver avant ou après l'installation du kit dans l'automate. Les poches 34 et 36 sont de préférence fournies remplies, respectivement de milieu de culture et de facteurs de croissance.

Tous les raccords des tubulures et des tubulures aux poches, ainsi que les moyens de prélèvement, font également de préférence partie d'un module pré-assemblé qui est schématiquement représenté en figure 5, les poches 34, 36 et 38 qui ne font pas nécessairement partie de ce module étant représentées en traits pointillés.

Les poches 36, 38, 40 et 56 ont une contenance de l'ordre de 150 ml environ, les poches 46 ont une contenance de 600 ml environ, et la poche 34 de milieu de culture a une contenance de l'ordre de 1000 ml environ.

Dans le cas où l'automate 10 est utilisé pour cultiver des cellules souches CD34+, la poche 38 comprend des cellules de ce type issues du prélèvement sur un patient et éventuellement isolées et purifiées, et les facteurs de croissance de la poche 36 sont des cytokines.

La poche d'expansion cellulaire 54 et la poche 56 formant piège à air sont portées par le dispositif d'agitation 16 et sont logées dans l'enceinte 14 de l'incubateur 12 (figure 3). Les autres poches 34, 36, 38, 40 et 46 et les moyens de prélèvement 62 sont situés à l'extérieur de l'enceinte 14.

Les tubulures 60, 66 et 70 de liaison de la poche d'expansion cellulaire 54 aux éléments situés à l'extérieur de l'enceinte 14 passent à travers un organe de l'incubateur, qui autorise la fermeture étanche de l'enceinte 14, cet organe étant représenté aux figures 3 et 6.

Cet organe est un élément de paroi formé par un bloc 80 de matière (par exemple plastique) qui est fixé sur le bord périphérique de l'ouverture de l'armoire de l'incubateur 12 et qui comprend trois gorges 82 parallèles d'engagement et de passage des tubulures 60, 66, 70 précitées. Ces gorges 82 sont sensiblement rectilignes et à distance les unes des autres. Le bloc 80 a une forme sensiblement plane et s'étend dans un plan vertical. Il comprend une face arrière en appui sur le bord périphérique de l'ouverture de l'armoire et une face avant sur laquelle sont formées les gorges 82, qui ont une orientation sensiblement horizontale et s'étendent sur toute la dimension transversale du bloc.

Les gorges 82 ont une section sensiblement circulaire et ont un diamètre interne légèrement supérieur à celui des tubulures 60, 66, 70. Ces tubulures sont destinées à être engagées complètement dans ces gorges et à passer éventuellement à travers des découpes 84 du joint périphérique 52 du bord de l'ouverture de l'armoire.

En position de fermeture de l'enceinte 14, le bord périphérique de la porte interne 48 est destiné à prendre appui sur le joint 52 et à recouvrir les parties des tubulures 60, 66, 70 s'étendant dans les découpes 84 précitées du joint 52, et le joint périphérique de la porte externe 50 est destiné à venir en appui sur la face avant du bloc et à recouvrir les gorges 82 et les parties des tubulures 60, 66, 70 s'étendant dans ces gorges.

Dans l'exemple représenté, la gorge inférieure du bloc 80 forme un passage de la tubulure 66 de liaison de la poche d'expansion cellulaire 54 aux poches de récupération 46, la gorge médiane forme un passage de la tubulure 60 de liaison de la poche 54 aux moyens de prélèvement 62, et la gorge supérieure forme un passage de la tubulure 70 de liaison de la poche 54 aux poches 34, 36, 38 et 40.

Comme cela est représenté en figure 3, la tubulure 70 est engagée dans la pompe 22 au voisinage de la poche 34 de milieu de culture, cette pompe étant une pompe péristaltique pour éviter les risques de contamination de ce milieu.

Les vannes 20 précitées sont des électrovannes qui sont au nombre de douze dans l'exemple représenté, référencées de 86 à 108 en figure 3.

Les tubulures 66, 60, 70 et 78 sont engagées respectivement dans quatre vannes 86, 88, 90 et 92 qui sont portées par le dispositif d'agitation 16 (figures 3 et 4).

La partie de la tubulure 70 située à l'extérieur de l'enceinte 14 est engagée dans deux vannes 94 et 96 à distance l'une de l'autre et dont une 94 est située à proximité de la poche 34 de milieu de culture. L'autre vanne 96 est située en aval de la connexion de la tubulure 70 aux tubulures 72 et en amont de la connexion de la tubulure 70 aux tubulures 74.

Les tubulures 72 reliées aux orifices d'entrée des poches 36 et 38 sont engagées dans des vannes 98 et 100, respectivement, et les tubulures 74 reliées aux orifices de sortie des poches 36 et 38 sont engagées dans des vannes 102 et 104, respectivement.

Les tubulures 76 reliées à la poche 40 formant piège à air sont chacune engagées dans une vanne 106, 108.

La pompe 22 et les vannes comprennent de préférence une gorge transversale de montage d'une tubulure par translation dans une direction perpendiculaire à l'axe longitudinal de la tubulure ou de la gorge.

Comme cela est schématiquement représenté en figure 3 et visible en figure 2, les poches 34, 36, 38, 40 et 56 et les tubulures 40, 72, 74, 76, 78 de liaison de ces poches à la poche d'expansion cellulaire 54 sont toutes situées au dessus de cette poche 54 lorsque cette dernière est disposée horizontalement. Les poches 46, les moyens de prélèvement 62 et les tubulures 60, 66 de liaison de ces éléments à la poche d'expansion cellulaire 54 sont tous situés au dessous de cette poche 54 lorsque cette dernière est disposée horizontalement.

Les poches 36, 38 et 56 sont sensiblement situées dans un même plan horizontal qui est situé en dessous d'un plan horizontal dans lequel sont situées les poches 34 et 40.

Le kit de consommables peut être installé dans l'automate de la façon suivante. Les portes 48, 50 de l'incubateur 12 sont ouvertes. Les poches 34, 36, 38, 40, 46 sont accrochées sur le bâti et la poche 56 est accrochée sur le bras du dispositif d'agitation 16. La poche 54 est posée à plat sur le dispositif d'agitation 16. La tubulure 70 est engagée dans les vannes 94, 96 ainsi que dans la pompe 22, les tubulures 72 sont engagées dans les vannes 98, 100, les tubulures 74 sont engagées dans les vannes 102, 104 et les tubulures 76 sont engagées dans les vannes 106, 108. Les tubulures 66, 60, 70 et 78 sont engagées respectivement dans les vannes 86, 88, 90 et 92 portées par le dispositif 16, puis les tubulures 66, 60 et 70 sont engagées respectivement dans les gorges 82 du bloc 80. Les tubulures sont connectées aux poches qui ne sont pas déjà pré-assemblées aux tubulures, puis les portes 48, 50 de l'incubateur 12 sont refermées.

On se réfère désormais aux figures 7 à 13 qui représente un mode de réalisation du dispositif d'agitation 16 selon l'invention.

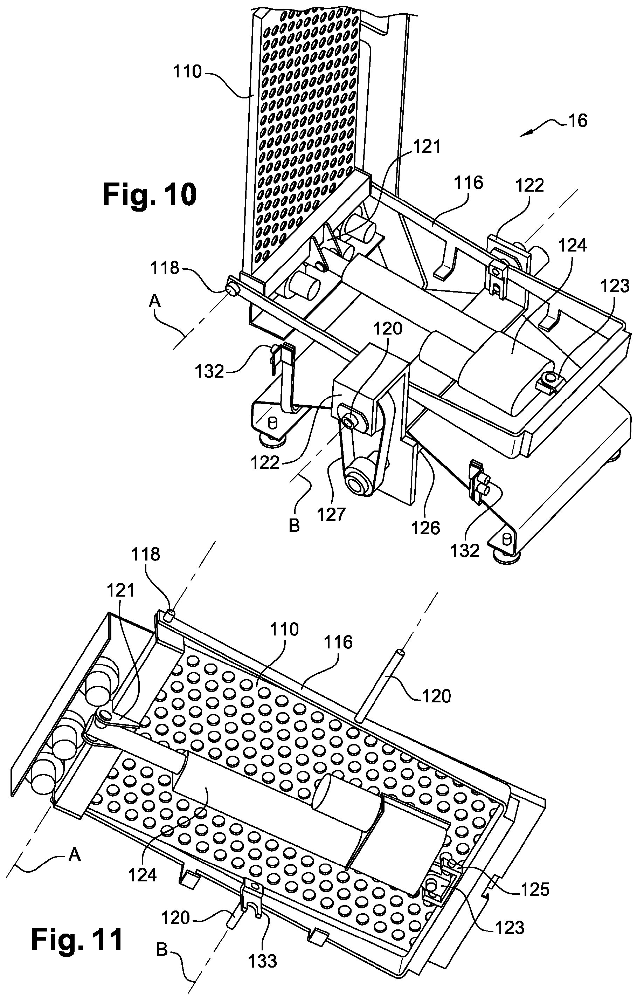

Le dispositif d'agitation 16 comprend un plateau 110 de support de la poche d'expansion cellulaire 54 (non représentée aux figures 7 à 13), ce plateau étant monté mobile en rotation autour d'un premier axe horizontal A pour le déplacement du plateau depuis une position sensiblement horizontale représentée en figures 7 et 11 jusqu'à une position sensiblement verticale représentée en figures 8 et 10 (le plateau 110 pouvant adopter n'importe quelle position entre ces positions extrêmes, telle qu'une position représentée en figure 9 dans laquelle il est incliné à 45° environ par rapport à un plan horizontal), et autour d'un second axe horizontal B autour duquel le plateau 110 est destiné à osciller (sur une plage angulaire de +/- 8° environ) pour agiter et homogénéiser le contenu de la poche d'expansion cellulaire.

Le plateau 110 a une forme rectangulaire dont les dimensions sont légèrement supérieures à celles de la poche d'expansion cellulaire 54 (de 40 cm environ de longueur et de 22 cm environ de largeur) qui est destinée à être posée à plat sur le plateau. Le plateau 110 comprend des rebords périphériques 112 de retenue de la poche et est perforé pour que la face de la poche 54 appuyée contre le plateau puisse être directement exposée au moins en partie aux conditions environnementales qui règnent dans l'enceinte 14 de l'incubateur 12.

Le plateau 110 comprend à l'une de ses extrémités, correspondant à l'un des petits côtés du plateau, un crochet 114 de fixation de la poche d'expansion cellulaire 54, ce crochet étant destiné à représenter le point le plus haut du dispositif 16 lorsque le plateau est en position verticale (figure 8). Le plateau 110 comprend à son extrémité opposée au crochet 114 trois orifices de montage des vannes 86, 88, 90 précitées.

Le dispositif 16 comprend une pièce 116 en U dont les extrémités libres des deux branches latérales sont articulées sur des pivots 118 fixés sur les bords latéraux de l'extrémité du plateau 110 portant les vannes 86, 88, 90. Ces pivots 118 sont alignés et définissent le premier axe A précité de rotation du plateau 110.

Les branches de la pièce 116 en U portent, sensiblement en leur milieu, des pivots 120 qui sont articulés sur un châssis 122 du dispositif 16, ces pivots 120 étant alignés et définissant le second axe B précité de rotation du plateau 110.

Lorsque le plateau 110 est en position sensiblement horizontale (figure 7), la pièce 116 en U s'étend le long de trois côtés du plateau, à savoir les côtés de plus grande longueur et le petit côté portant le crochet 114.

Le déplacement du plateau 110 autour de l'axe A est assuré par un vérin 124 qui est monté entre les branches de la pièce 116 en U et dont le cylindre est fixé à la partie médiane de cette pièce 116 et la tige de piston est fixée à l'extrémité du plateau portant les vannes 86, 88 et 90.

Comme cela est visible aux figures 10 et 11, la tige de piston du vérin 124 est articulée sur un axe porté par une chape 121 fixée sur l'extrémité du plateau 110 portant les vannes, cet axe étant sensiblement horizontal. Le cylindre du vérin 124 est articulé sur un axe sensiblement vertical porté par une première chape 123 qui est elle-même articulée sur un axe sensiblement horizontal porté par une deuxième chape 125, cette deuxième chape 125 étant fixée sur la partie médiane de la pièce 116, sensiblement en son milieu.

Lorsque la tige de piston du vérin 124 est en position sortie, le plateau 110 est dans sa position sensiblement horizontale représentée aux figures 7 et 11. Lorsque la tige de piston du vérin 124 est en position complètement rentrée, le plateau 110 est dans sa position sensiblement verticale représentée aux figures 8 et 10. Dans le cas de la figure 9, la tige de piston du vérin 124 est partiellement rentrée ou sortie.

Le déplacement du plateau autour de l'axe B est assuré par un moteur électrique 126 dont l'arbre de sortie entraîne par l'intermédiaire d'une courroie 127 une roue d'entraînement de l'un des pivots 120 portés par la pièce 116 en U (figure 10). Le moteur 126 est fixé au châssis 122 du dispositif par des moyens appropriés.

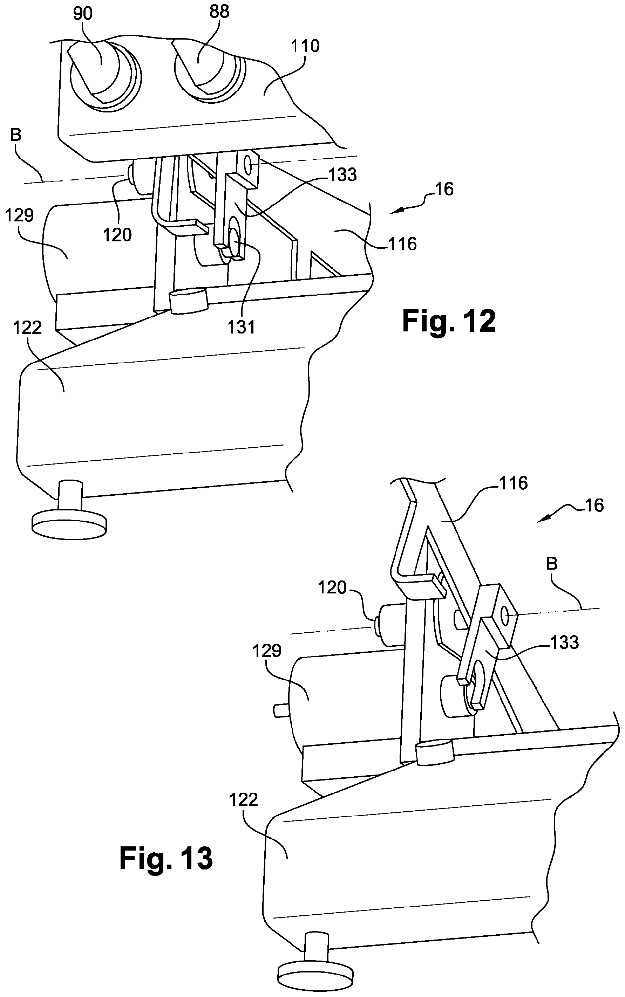

Comme cela est représenté aux figures 12 et 13, le châssis 122 du dispositif d'agitation 16 porte un système 129 de blocage de la rotation du plateau 110 autour de l'axe B, ce système 129 comportant un doigt rétractable 131 qui coopère avec un élément 133 porté par la pièce 116 en U pour le blocage du plateau.

Le doigt 131 est déplaçable depuis une position sortie représentée en figure 12 jusqu'à une position rentrée représentée en figure 13, le déplacement de ce doigt étant commandé par le système informatique 24.

L'élément 133 porté par la pièce 116 en U a une forme allongée et comprend une première extrémité solidaire de l'un des pivots 120 de la pièce et une seconde extrémité comportant une encoche dans laquelle est destiné à être engagé le doigt 131 pour bloquer la rotation du plateau 110 autour de l'axe B. Lorsque le doigt 131 est déployé (figure 12), les faces latérales de l'encoche de l'élément 133 peuvent venir en butée sur le doigt empêchant ainsi toute rotation du plateau autour de l'axe B. Lorsque le doigt est en position rentrée (figure 13), la pièce 116 et le plateau 110 peuvent être déplacés en rotation autour de l'axe B.

Le blocage de la rotation du plateau 110 autour de l'axe B peut être activé par le système informatique 24 lorsque le plateau est déplacé autour de l'axe A jusqu'à une position inclinée ou verticale, en vue d'un prélèvement ou de la récupération des cellules de la poche d'expansion cellulaire 54, pour éviter que le plateau ne se déplace autour de l'axe B du fait de la force exercée d'un côté du plateau par le poids de la poche d'expansion cellulaire.

Le dispositif d'agitation 16 comprend également un bras vertical 128 de fixation de la vanne 92 précitée et d'accrochage de la poche 56 formant piège à air. La vanne 92 est située sensiblement à mi-hauteur du bras 128 et l'extrémité supérieure du bras comprend un crochet 130 de fixation de la poche 56 (figures 7 à 9).

Le dispositif 16 comprend en outre des capteurs 132 de position du plateau 110 autour des axes A et/ou B, qui sont portés par le châssis 122.

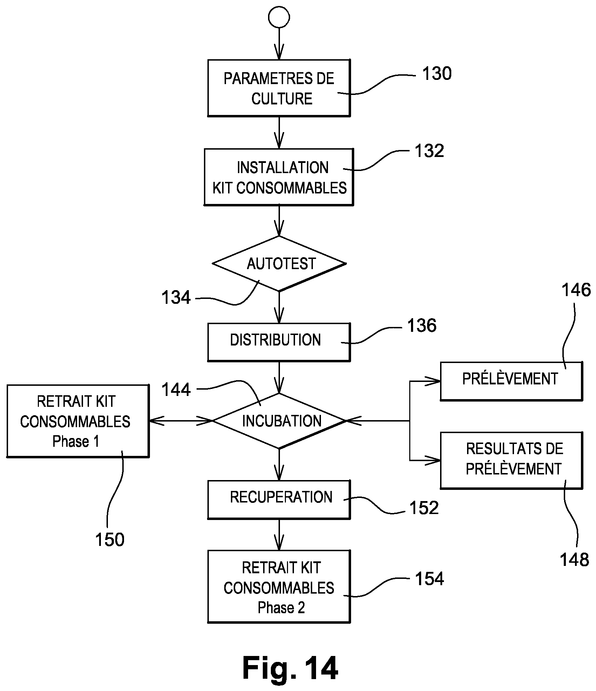

La figure 14 est un organigramme représentant des étapes du procédé selon l'invention.

Une première étape 130 du procédé consiste à saisir et enregistrer au moyen du système informatique 24 des paramètres de culture propres au protocole biologique. La saisie est réalisée par un opérateur, les paramètres saisis étant par exemple l'identification du patient, l'identification du kit de consommables, le volume de la poche d'expansion cellulaire 54, etc. Pour faciliter la saisie de ces paramètres, le système informatique 24 peut être équipé d'un lecteur de codes barres, le kit de consommables pouvant comprendre un code barres renseignant directement le système informatique 24 avec le numéro et la nature du kit ainsi que le volume de chaque poche.

Le procédé comprend une seconde étape 132 d'installation du kit de consommables dans l'automate 10, comme cela est décrit dans ce qui précède. Cette installation peut être guidée et supervisée par le système informatique 24. L'installation peut se faire en plusieurs sous-étapes, le système informatique 24 affichant des consignes d'installation à l'opérateur en lui indiquant de valider ou d'invalider la réalisation d'une sous-étape et le passage à la sous-étape suivante. Ces sous-étapes sont par exemple :
- le placement des différentes poches sur le bâti 18 et dans l'enceinte 14 de l'incubateur 12,
- le placement des tubulures 70 de la poche 34 du milieu de culture dans les vannes 94, 96 (le système informatique 24 commande alors l'ouverture des vannes 94, 96 qui sont ensuite fermées dès que l'opérateur a validé cette sous-étape),
- le placement des tubulures 72, 74, 76 de la poche 36 de facteurs de croissance et de la poche 40 formant piège à air dans les vannes 98, 102 et 106 (le système informatique 24 commande l'ouverture de ces vannes qui sont ensuite fermées dès que l'opérateur a validé cette sous-étape),
- le placement des tubulures 72, 74, 76 de la poche 38 de cellules à cultiver et de la poche 40 formant piège à air dans les vannes 100, 104 et 108 (le système informatique 24 commande l'ouverture de ces vannes qui sont ensuite fermées dès que l'opérateur a validé cette sous-étape),
- le placement de la tubulure 78 de la poche 56 formant piège à air dans la vanne 92 (le système informatique 24 commande l'ouverture de cette vanne qui est ensuite fermée dès que l'opérateur a validé cette sous-étape), et
- le placement des tubulures 70, 60, 66, les unes après les autres, dans les vannes 90, 88 et 86 (le système informatique 24 commande l'ouverture de chacune de ces vannes, les unes après les autres, qui sont ensuite fermées dès que l'opérateur a validé chaque sous-étape).

Le procédé selon l'invention comprend une troisième étape 134 de test appelée « autotest » lors de laquelle le système informatique 24 contrôle le bon fonctionnement des vannes ainsi que des moyens de basculement (vérin 124 et moteur 126) du plateau 110 du dispositif d'agitation 16. Le fonctionnement de l'incubateur 12 peut être implicitement contrôlé à l'initiation du protocole biologique, ce dernier ne pouvant être initié que si la température et le taux de CO₂, par exemple, dans l'enceinte 14 sont stabilisés aux valeurs de consigne d'incubation.

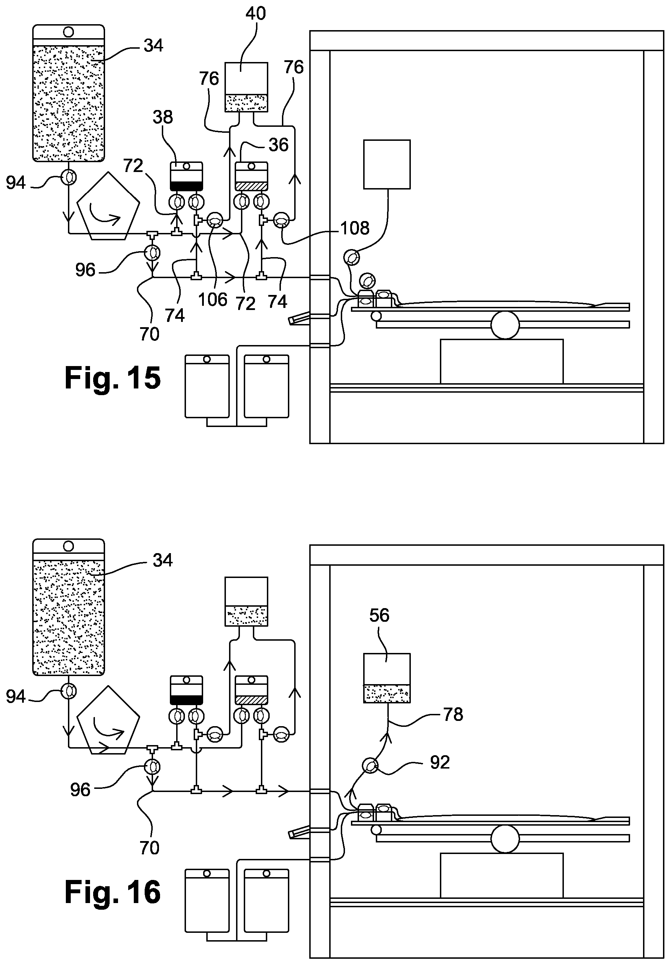

Le procédé selon l'invention comprend une autre étape 136 de distribution des fluides, qui comprend plusieurs sous-étapes schématiquement représentées aux figures 15 à 19.

La première sous-étape de l'étape 136 de distribution est représentée en figure 15 et consiste à évacuer l'air contenu dans les tubulures 70, 72, 74. Pour cela, les vannes 94, 96, 106 et 108 sont ouvertes et la pompe 22 est actionnée par le système informatique 24 pour que du milieu de culture circule depuis la poche 34 dans les tubulures 70, 72, 74 jusqu'à la poche 40 formant piège à air. Les tubulures 70, 72, 74 se remplissent alors de milieu de culture et la poche 40 se remplit au moins partiellement de milieu de culture. La pompe 22 est réglée à un débit prédéterminé et fonctionne pendant une durée prédéterminée, à la fin de laquelle la pompe est arrêtée et les vannes 94, 96, 106 et 108 sont fermées.

La seconde sous-étape de l'étape 136 de distribution est représentée en figure 16 et consiste à évacuer l'air contenu dans les tubulures 70 et 78. Pour cela, les vannes 94, 96 et 92 sont ouvertes et la pompe 22 est actionnée par le système informatique 24 pour que du milieu de culture circule depuis la poche 34 dans les tubulures 70, 78 jusqu'à la poche 56 formant piège à air. La tubulure 78 se remplit alors de milieu de culture et la poche 56 se remplit au moins partiellement de milieu de culture. La pompe 22 est réglée à un débit prédéterminé et fonctionne pendant une durée prédéterminée, à la fin de laquelle la pompe est arrêtée et les vannes 94, 96 et 92 sont fermées.

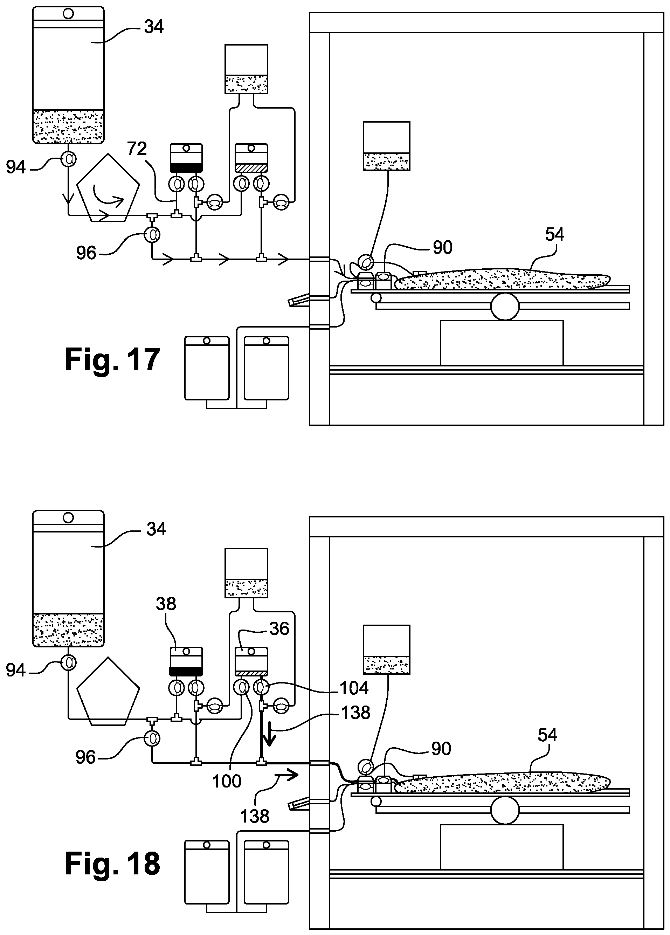

La troisième sous-étape de l'étape 136 de distribution est représentée en figure 17 et consiste à alimenter la poche d'expansion cellulaire 54 en milieu de culture. Les vannes 94, 96 et 90 sont ouvertes et la pompe 22 est actionnée par le système informatique 24 pour que du milieu de culture circule depuis la poche 34 jusqu'à la poche 54. La poche 54 se remplit alors de milieu de culture. La pompe 22 est réglée à un débit prédéterminé et fonctionne pendant une durée prédéterminé selon les paramètres du protocole biologique spécifiant le volume de milieu de culture à distribuer à la poche 54 ainsi que son débit d'alimentation. La pompe 22 est ensuite arrêtée et les vannes 94, 96, 90 sont fermées.

La quatrième sous-étape de l'étape 136 de distribution est représentée en figure 18 et consiste à alimenter la poche d'expansion cellulaire 54 en facteurs de croissance puis à rincer la poche 36 de facteurs de croissance avec du milieu de culture et à évacuer le contenu de cette poche 36 vers la poche d'expansion cellulaire 54. Dans un premier temps, les vannes 104 et 90 sont ouvertes, de façon à de que le milieu contenant les facteurs de croissance s'écoule par gravité depuis la poche 36 jusqu'à la poche 54 en circulant dans les tubulures 74 et 70 (flèches 138). La poche 54 se remplit en facteurs de croissance. Les vannes 104 et 90 sont ouvertes pendant une durée prédéterminée en fonction du volume de milieu contenant les facteurs de croissance à distribuer à la poche 54. A l'issue de cette durée, les vannes 104 et 90 sont fermées. Les vannes 94 et 100 sont ensuite ouvertes et la pompe 22 est actionnée (selon un débit et une durée prédéterminés) pour alimenter la poche 36 en milieu de culture en vue de son rinçage. Les vannes 94 et 100 sont fermées et les vannes 104 et 90 sont à nouveau ouvertes pour que le produit de rinçage contenu dans la poche 36 s'écoule par gravité jusqu'à la poche d'expansion cellulaire 54. Les vannes 104 et 90 sont ouvertes pendant une durée prédéterminée en fonction du volume de ce produit de rinçage à distribuer à la poche 54. A l'issue de cette durée, les vannes 104 et 90 sont refermées. Ces phases de rinçage de la poche 36 et d'évacuation du produit de rinçage vers la poche d'expansion cellulaire 54 peuvent être répétées une ou plusieurs fois selon les paramètres du protocole biologique, afin par exemple que l'intégralité des facteurs de croissance contenus à l'origine dans la poche 36 se retrouvent dans la poche 54.

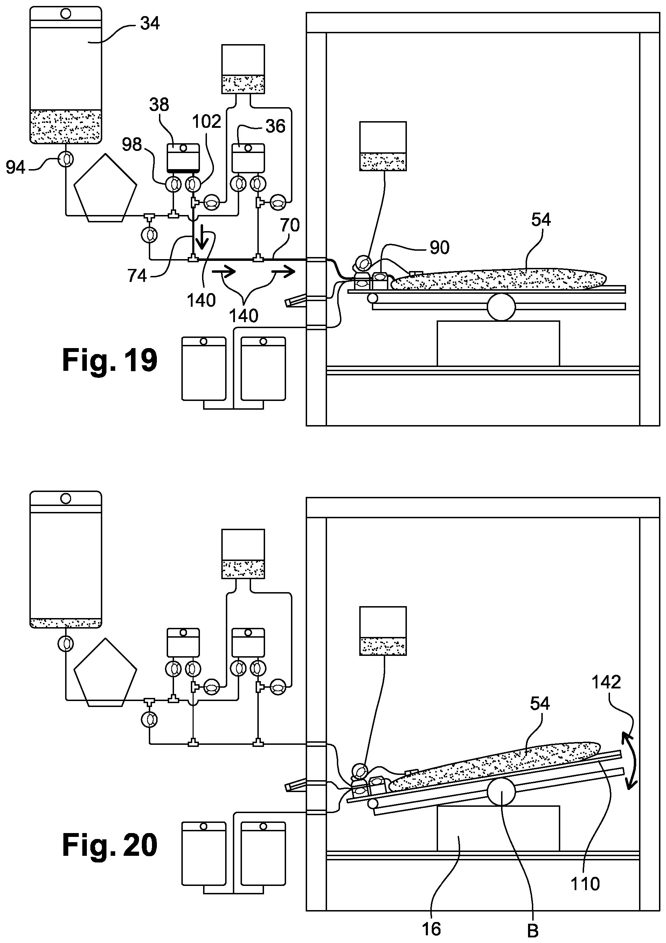

La cinquième sous-étape de l'étape 136 de distribution est représentée en figure 19 et consiste à alimenter la poche d'expansion cellulaire 54 en cellules à cultiver puis à rincer la poche 38 contenant ces cellules avec du milieu de culture et à évacuer le contenu de cette poche 38 vers la poche d'expansion cellulaire 54, d'une façon similaire à celle réalisée lors de la quatrième sous-étape. Dans un premier temps, les vannes 102 et 90 sont ouvertes, de façon à ce que le milieu contenant les cellules à cultiver s'écoule par gravité depuis la poche 38 jusqu'à la poche 54 en circulant dans les tubulures 74 et 70 (flèches 140). La poche 54 se remplit en cellules à cultiver. Les vannes 102 et 90 sont ensuite fermées et les vannes 94 et 98 sont ouvertes et la pompe 22 est actionnée (selon un débit et une durée prédéterminés) pour alimenter la poche 38 en milieu de culture en vue de son rinçage. Les vannes 94 et 98 sont fermées et les vannes 102 et 90 sont à nouveau ouvertes pour que le produit de rinçage contenu dans la poche 38 s'écoule par gravité jusqu'à la poche d'expansion cellulaire 54. Les vannes 102 et 90 sont ensuite fermées. Ces phases de rinçage de la poche 38 et d'évacuation du produit de rinçage vers la poche d'expansion cellulaire 54 peuvent être répétées une ou plusieurs fois selon les paramètres du protocole biologique, afin par exemple que toutes les cellules à cultiver contenues à l'origine dans la poche 36 se retrouvent dans la poche 54.

L'étape de distribution 136 du procédé peut être suivie d'une étape d'homogénéisation du contenu de la poche d'expansion cellulaire 54, qui est schématiquement représentée en figure 20. Lors de cette étape, le système informatique 24 commande le dispositif d'agitation 16 de façon à ce que le plateau 110 oscille autour de l'axe B, comme expliqué dans ce qui précède (flèches 142). L'amplitude, la fréquence, la durée et les périodes (agitation repos, agitation, etc.) de ces oscillations sont déterminées en fonction des paramètres du protocole biologique.

Le procédé selon l'invention comprend ensuite une étape d'incubation 144 qui peut durer plusieurs jours et par exemple une dizaine de jours. Périodiquement, selon les paramètres du protocole, le contenu de la poche d'expansion cellulaire 54 peut être homogénéisé, en déplaçant en rotation le plateau autour de l'axe B comme expliqué ci-dessus. Cette homogénéisation (périodes, fréquence, amplitude) est déterminée par les paramètres du protocole de façon indépendante de l'étape d'homogénéisation suivant l'étape de distribution 136.

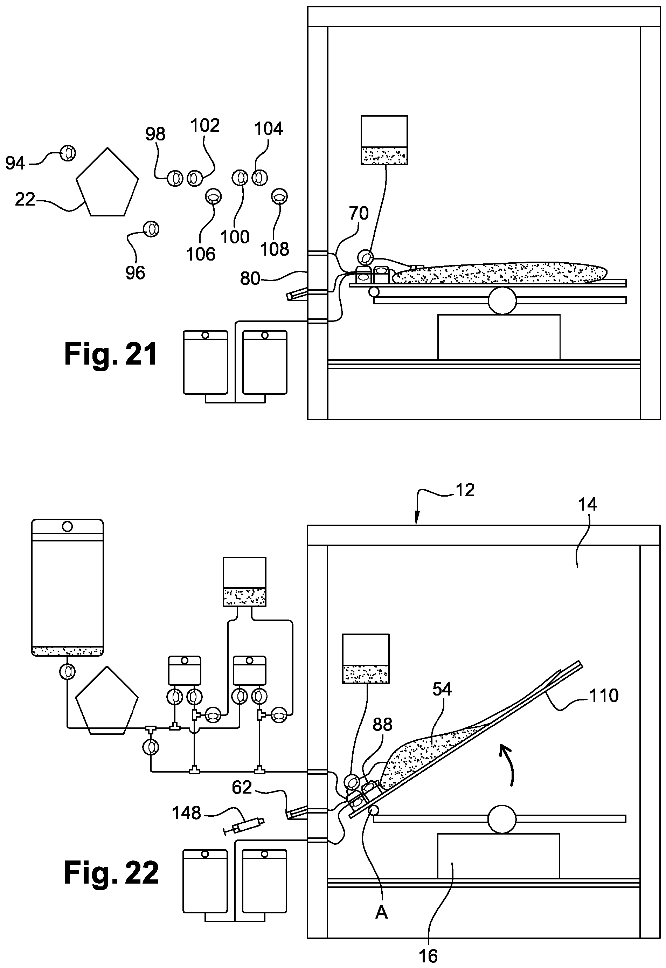

Pendant l'étape d'incubation 144, l'opérateur peut effectuer un ou plusieurs prélèvements 146 dans la poche d'expansion cellulaire 54 (figures 14 et 22). Certains de ces prélèvements peuvent être imposés par le système informatique. Ces prélèvements obligatoires sont par exemple au nombre de trois et peuvent être effectués juste après l'étape de distribution, trois jours après le début de l'étape d'incubation 144, et sept jours après le début de cette étape 144. D'autres prélèvements peuvent être réalisés à la volonté de l'opérateur, le système informatique pouvant inviter l'opérateur à réaliser ces prélèvements facultatifs.

Lorsque l'opérateur confirme au système informatique 24 qu'il est prêt à effectuer un prélèvement, le système informatique actionne le vérin 124 pour que le plateau 110 du dispositif d'agitation 16 soit déplacé en rotation autour de l'axe A jusqu'à une position inclinée, par exemple de 45° environ, par rapport à un plan horizontal, comme cela est schématiquement représenté en figure 9 et 22. Le système informatique 24 peut alors détecter la bonne position du plateau 110 via les capteurs du dispositif 16.

Le système informatique 24 commande l'ouverture de la vanne 88 de façon à ce qu'une partie du contenu de la poche d'expansion cellulaire 54 s'écoule par gravité depuis la poche 54 dans la tubulure 60 jusqu'aux moyens de prélèvement 62 situés à l'extérieur de l'enceinte 14 de l'incubateur 12. L'opérateur peut effecteur le prélèvement d'un échantillon de la poche d'expansion cellulaire 54 par l'intermédiaire d'une seringue 148 équipée de moyens de connexion du type « Luer lock » qui sont engagés dans les moyens de prélèvement 62. Après le prélèvement, la vanne 88 est fermée et le plateau 110 du dispositif d'agitation 16 est ramené dans une position sensiblement horizontale.

L'opérateur peut alors procéder à des analyses de l'échantillon prélevé, les résultats 148 de ces analyses pouvant être saisis et enregistrés dans le système informatique 24 par l'opérateur.

Pendant l'étape d'incubation 144, l'opérateur peut également retirer une partie du kit de consommables (première phase de retrait 150 des consommables - figures 14 et 21). Les éléments du kit de consommables qui peuvent être retirés sont toutes les poches (34, 36, 38, 40) et tubulures (72, 74, 76) reliées à la tubulure 70. Pour cela, l'opérateur doit couper la tubulure 70 en amont de sa traversée du bloc 80 précité et doit en même temps souder ou pincer l'extrémité libre coupée de la tubulure 70 restant dans l'enceinte 14 de l'incubateur 12, pour éviter toute contamination de la poche d'expansion cellulaire. Cette opération peut être réalisée par l'opérateur au moyen d'une pince coupante appropriée réalisant la fermeture étanche de l'extrémité de la tubulure lors de sa coupe. Les vannes 94 à 108 sont alors ouvertes pour autoriser le retrait par l'opérateur des tubulures 70, 72, 74, 76 de ces vannes ainsi que de la pompe 22 (figure 21). Une fois que l'opérateur a confirmé le retrait de ces éléments au système informatique 24, ce dernier commande la fermeture des vannes 94 à 108.

Le procédé selon l'invention comprend en outre une étape de récupération 152 des cellules après culture (figures 14, 23 et 24). A l'issue de l'étape d'incubation 144 et sur demande de l'opérateur, le système informatique 24 bloque la rotation du plateau 110 autour de l'axe B et actionne le vérin 124 pour que le plateau 110 du dispositif d'agitation 16 se déplace autour de l'axe A jusqu'à une position sensiblement verticale représentée aux figures 8, 23 et 24. Le système informatique 24 peut alors détecter la bonne position du plateau 110 via les capteurs du dispositif 16.

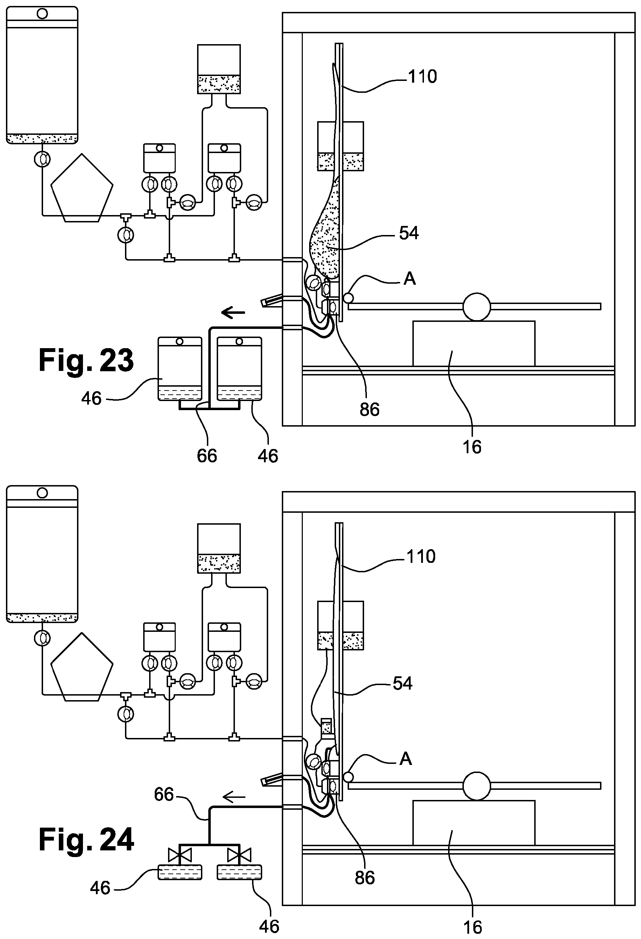

Le système informatique 24 commande ensuite l'ouverture de la vanne 86 de façon à ce que le contenu de la poche d'expansion cellulaire 54 s'écoule par gravité depuis la poche 54 dans les deux poches de récupération 46 en circulant dans la tubulure 66 (figure 23).

Les plaques 42 portées par le bâti 18 et sur lesquelles sont accrochées les poches de récupération 46 peuvent être déplacées depuis leur position verticale représentée aux figures 2 et 23 à leur position horizontale représentée schématiquement en figure 24, soit manuellement par l'opérateur soit par l'intermédiaire de moyens de déplacement commandés par le système informatique 24. Le basculement des plaques 46 permet aux poches 46 d'être entièrement situées en dessous de la poche 54 et d'au moins une partie de la tubulure 66 de façon à ce que le contenu de la poche d'expansion cellulaire 54 soit si possible intégralement transféré dans les poches de récupération 46. Lorsque l'opérateur a confirmé au système informatique 24 que le prélèvement est terminé, ce système commande la fermeture de la vanne 86 et le déploiement du vérin de façon à ce que le plateau 110 revienne en position sensiblement horizontale.

Les poches 46 sont alors retirées de l'automate 10 en vue d'un traitement éventuel des cellules et de la réinjection de ces cellules dans le corps d'un patient pour une thérapie cellulaire par exemple. Pour cela, la tubulure 66 peut être coupée et soudée par la pince précitée ou les poches 46 sont déconnectées de la tubulure 66.

La dernière étape du procédé consiste en une seconde phase de retrait 154 des consommables de l'automate 10, les poches 54, 56 et les tubulures restantes 66, 60, 70 et 78 étant retirées. Pour cela, le système informatique 24 commande l'ouverture des vannes 86, 88, 90 et 92 pour autoriser le retrait par l'opérateur des tubulures 66, 60, 70 et 78. Une fois que l'opérateur a confirmé le retrait de ces éléments au système informatique 24, ce dernier commande la fermeture des vannes 86, 88, 90 et 92.

Lorsque le protocole biologique est terminé, le système informatique peut éditer un rapport de culture, ce rapport pouvant comprendre les informations suivantes pour assurer une bonne traçabilité du protocole : des informations propres au constructeur (le numéro d'identification de l'automate, la version du logiciel de fonctionnement du système informatique, la version du logiciel de supervision du protocole biologique), l'ensemble des paramètres du protocole dès lors que l'un au moins de ces paramètres n'est pas à sa valeur par défaut, l'ensemble des paramètres de culture, les actions effectuées par l'opérateur (comprenant la date de chaque action au format aaaammjjThhmiss, l'auteur de cette action via un identifiant de connexion, la nature de l'action via une codification à définir à défaut d'un libellé équivoque), des événements « système » (comprenant la date de l'événement au format aaaammjjThhmiss, la nature de l'événement (alarme, alerte utilisateur, détection de défaillance, etc.) via une codification à définir à défaut d'un libellé équivoque), des résultats d'analyse de prélèvements (comprenant la date de chaque prélèvement, l'auteur du prélèvement, les résultats d'analyse du prélèvement, etc.), et des informations du greffon (issues de l'analyse des cellules récupérées après culture). Ce rapport de culture peut être accessible par le poste informatique du réseau précité.

## Revendications

1. Automate (10) de culture cellulaire, comprenant des réservoirs (34, 36, 38) de milieu de culture, de facteurs de croissance et de cellules à cultiver, un incubateur (12) à enceinte (14) thermostatée dans laquelle est logé un récipient (54) de culture des cellules ou d'expansion cellulaire, et un système informatique (24) de commande incluant des moyens de saisie et d'enregistrement de données et destiné à réguler les conditions de culture dans l'enceinte et à piloter des vannes (20) de distribution de fluides selon une séquence prédéfinie, **caractérisé en ce qu'**il comprend un dispositif (16) de support et d'agitation du récipient de culture ou d'expansion cellulaire qui est commandé par le système informatique et qui est logé dans l'enceinte, et **en ce que** le récipient est formé par une poche d'expansion cellulaire (54) comportant au moins un orifice d'entrée (68) relié aux réservoirs précités et un orifice de sortie (64) relié à des moyens (46) de récupération et de stockage des cellules après culture, ces moyens de stockage et les réservoirs étant situés à l'extérieur de l'enceinte et étant reliés aux orifices de la poche d'expansion cellulaire par des conduits (66, 70, 72, 74) qui forment avec la poche d'expansion cellulaire un module pré-assemblé posé dans l'enceinte et qui traversent un élément de paroi de l'enceinte, de façon à permettre d'alimenter la poche d'expansion cellulaire en milieu de culture, en facteurs de croissance et en cellules à cultiver, et de récupérer le contenu de la poche d'expansion cellulaire dans les moyens de stockage, en maintenant l'enceinte fermée, le dispositif (16) de support et d'agitation comprenant un plateau (110) de support de la poche d'expansion cellulaire (54), qui est monté rotatif autour d'un premier axe horizontal (A) et qui est déplaçable autour de cet axe entre une position sensiblement horizontale de culture des cellules et une position sensiblement verticale de récupération des cellules après culture.

2. Automate selon la revendication 1, **caractérisé en ce que** la poche d'expansion cellulaire (54) comprend en outre un orifice de prélèvement (58) qui est relié par un conduit (60) à des moyens de prélèvement (62) situés à l'extérieur de l'enceinte (14), ce conduit traversant la partie précitée de l'incubateur et faisant partie du module pré-assemblé.

3. Automate selon la revendication 1 ou 2, **caractérisé en ce que** l'incubateur (12) comprend une armoire comportant une ouverture et équipée d'une porte (50) de fermeture étanche de cette ouverture, des moyens (80) de passage des conduits (60, 66, 70) précités étant montés sur le bord périphérique de cette ouverture et comportant des gorges (82) sensiblement parallèles d'engagement des conduits, ces gorges étant destinées à être recouvertes par la porte lorsqu'elle est en position fermée.

4. Automate selon l'une des revendications précédentes, **caractérisé en ce que** les réservoirs de facteurs de croissance et de cellules à cultiver sont formés par des poches (36, 38) qui sont situées plus haut que l'orifice d'entrée (68) de la poche d'expansion cellulaire (54) de façon à ce que le contenu de chacune des poches de facteurs de croissance et de cellules à cultiver puisse s'écouler par gravité jusqu'à la poche d'expansion cellulaire.

5. Automate selon l'une des revendications précédentes, **caractérisé en ce que** les moyens de stockage comprennent une ou deux poches (46) qui sont au moins en partie situées plus bas que l'orifice de sortie (64) de la poche d'expansion cellulaire (54) de façon à ce que, après culture, le contenu de la poche d'expansion cellulaire puisse s'écouler par gravité jusqu'à la ou les poches des moyens de stockage.

6. Automate selon l'une des revendications précédentes, **caractérisé en ce que** la poche d'expansion cellulaire (54) comprend des parois souples étanches aux liquides et perméables aux gaz, en particulier au CO₂, et ayant de préférence des propriétés limitant au maximum l'adhérence des cellules à cultiver avec les parois de la poche.

7. Automate selon l'une des revendications précédentes, **caractérisé en ce qu'**il comprend une pompe péristaltique (22) de commande de l'alimentation en milieu de culture de la poche d'expansion cellulaire (54) et des réservoirs (36, 38) de facteurs de croissance et de cellules à cultiver, en vue du rinçage de ces réservoirs.

8. Automate selon l'une des revendications précédentes, **caractérisé en ce qu'**il comprend deux poches (40, 56) formant piège à air, dont l'une est reliée aux réservoirs (36, 38) de facteurs de croissance et de cellules à cultiver, et dont l'autre est reliée à la poche d'expansion cellulaire (54), et qui sont destinées à collecter et stocker l'air contenu dans les conduits (70, 72, 74), la poche d'expansion cellulaire (54) et/ou les réservoirs (36, 38).

9. Automate selon l'une des revendications précédentes, **caractérisé en ce que** les conduits sont formés par des tubulures souples (60, 66, 70, 72, 74, 76, 78), dont au moins certaines traversent des vannes (86, 88, 90, 92, 94, 96, 98, 100, 102, 104, 106, 108) qui sont destinées, en position de fermeture, à pincer ces tubulures.

10. Automate selon l'une des revendications 1 à 9, **caractérisé en ce que** le plateau (110) est monté rotatif autour d'un second axe horizontal (B) autour duquel le plateau est destiné à osciller pour l'agitation et l'homogénéisation du contenu de la poche d'expansion cellulaire.

11. Automate selon l'une des revendications 1 à 10, **caractérisé en ce que** le plateau (110) porte des vannes (86, 88, 90) de commande de l'alimentation de la poche d'expansion cellulaire (54), de récupération du contenu de cette poche, et de prélèvement d'échantillons de cette poche.

12. Automate selon l'une des revendications 1 à 11, **caractérisé en ce que** le dispositif (16) de support et d'agitation comprend un bras vertical (128) comportant à son extrémité supérieure des moyens (130) d'accrochage d'une poche (56) formant piège à air reliée à la poche d'expansion cellulaire (54).

13. Automate selon l'une des revendications précédentes, **caractérisé en ce que** les moyens de stockage (46) sont montés rotatifs autour d'un axe horizontal et sont déplaçables autour de cet axe entre une position sensiblement verticale et une position sensiblement horizontale dans laquelle ces moyens sont entièrement situés en dessous de la poche d'expansion cellulaire.

14. Kit de culture cellulaire stérile et à usage unique pour un automate (10) de culture cellulaire , **caractérisé en ce qu'**il comprend au moins une poche (54) de culture et des tubulures souples (60, 66, 70, 72, 74) de liaison de la poche à d'autres poches ou réservoirs, les tubulures et la poche d'expansion cellulaire formant un module pré-assemblé, la poche étant destinée à être posée dans une enceinte de culture cellulaire et les tubulures étant destinées à traverser un élément de paroi de ladite enceinte, la poche d'expansion cellulaire comprenant un orifice d'entrée (68), un orifice de sortie (64) et éventuellement un orifice de prélèvement (58), l'orifice d'entrée (68) de la poche d'expansion cellulaire (54) étant relié par des tubulures (70, 72, 74) à des orifices d'entrée et de sortie d'une poche (36) de facteurs de croissance, et à des orifices d'entrée et de sortie d'une poche (38) de cellules à cultiver, le kit comprenant en outre deux poches (40, 56) formant piège à air dont l'une est reliée aux orifices de sortie des poches (36, 38) de facteurs de croissance et de cellules à cultiver, et dont l'autre est reliée à l'orifice d'entrée (68) de la poche d'expansion cellulaire (54).

15. Kit selon la revendication 14, **caractérisé en ce qu'**il comprend une ou deux poches (46) de récupération des cellules après culture, qui sont reliées par des tubulures (66) à l'orifice de sortie (64) de la poche d'expansion cellulaire (54).

16. Automate selon l'une des revendications 1 à 13 comprenant un dispositif (16) de support et d'agitation pour un automate (10) de culture cellulaire, **caractérisé en ce qu'**il comprend un plateau (110) de support d'une poche d'expansion cellulaire (54), ce plateau portant trois vannes (86, 88, 90) et étant monté en rotation autour d'un premier axe horizontal (A) pour le basculement du plateau depuis une position sensiblement horizontale jusqu'à une position sensiblement verticale, et un second axe horizontal (B) autour duquel le plateau est destiné à osciller pour l'agitation et l'homogénéisation du contenu de la poche d'expansion cellulaire, le dispositif comprenant également des moyens (124, 126) commandés de basculement du plateau autour des axes horizontaux précités.

17. Automate selon la revendication 16, **caractérisé en ce qu'**il comprend un bras vertical (128) comportant à son extrémité supérieure des moyens (130) d'accrochage d'une poche (56) formant piège à air.

18. Procédé automatisé de culture cellulaire au moyen d'un automate (10) selon l'une des revendications 1 à 13, 16 et 17, **caractérisé en ce qu'**il comprend les étapes consistant à :
a) alimenter la poche d'expansion cellulaire (54) en milieu de culture, en facteurs de croissance puis en cellules à cultiver, en maintenant l'enceinte (14) de l'incubateur (12) fermée ;
b) agiter la poche d'expansion cellulaire en vue de l'homogénéisation de son contenu ;
c) maintenir la poche d'expansion cellulaire dans des conditions d'incubation pendant une durée de plusieurs jours ; et
d) récupérer le contenu de la poche d'expansion cellulaire dans les moyens de stockage (46), en maintenant l'enceinte fermée.

19. Procédé selon la revendication 18, **caractérisé en ce qu'**il comprend :
- avant l'étape a), une étape d'installation du module pré-assemblé en montant la poche d'expansion cellulaire (54) sur le dispositif d'agitation (16), en montant les conduits (60, 66, 70, 72, 74) dans les moyens de passage (80) de l'incubateur (12) et dans les vannes (20), et en connectant ces conduits aux réservoirs ou poches (34, 36, 38), et/ou
- avant l'étape a), une étape d'évacuation de l'air contenu dans les conduits (70, 72, 74) par passage de milieu de culture depuis le réservoir (34) de milieu de culture jusqu'à la ou les poches (40, 56) formant piège à air ; et/ou
- après l'alimentation de la poche d'expansion cellulaire en facteurs de croissance à l'étape a), une étape de rinçage du réservoir (36) de facteurs de croissance en faisant passer du milieu de culture dans ce réservoir puis en évacuant son contenu jusqu'à la poche d'expansion cellulaire (54) ; et/ou
- après l'alimentation de la poche d'expansion cellulaire en cellules à cultiver à l'étape a), une étape de rinçage du réservoir (38) de cellules à cultiver en faisant passer du milieu de culture dans ce réservoir puis en évacuant son contenu jusqu'à la poche d'expansion cellulaire (54) ; et/ou
- pendant l'étape c), une ou plusieurs étapes de prélèvement d'échantillon du contenu de la poche d'expansion cellulaire (54), qui sont chacune précédées d'une étape de basculement du plateau de support (110) d'une position horizontale de culture jusqu'à une position inclinée dans laquelle l'orifice de prélèvement (58) de la poche représente le point le plus bas de la poche ; et/ou
- avant l'étape c), une étape de retrait des réservoirs (34, 36, 38) de milieu de culture, de facteurs de croissance et de cellules à cultiver, en coupant et en soudant ou pinçant le conduit ou la tubulure (70) de liaison de ces réservoirs à l'orifice d'entrée (68) de la poche d'expansion cellulaire (54) ; et/ou
- avant ou pendant l'étape d), à basculer le plateau (110) dans une position sensiblement verticale de façon à ce que l'orifice de sortie (64) de la poche d'expansion cellulaire (54) représente le point le plus bas de la poche.

20. Utilisation d'un automate selon l'une des revendications 1 à 13, 16, 17 ou d'un kit selon la revendication 14 ou 15 pour la culture de cellules souches du type CD34+ ou de cellules mononuclées du sang, telles que par exemple des lymphocytes.

21. Utilisation selon la revendication 20, **caractérisée en ce que** les cellules souches proviennent d'une ou plusieurs sources comme plus particulièrement, le sang de cordon ombilical, la moelle osseuse et le sang total.

## Patentansprüche

1. Automat (10) zur Zellkultivierung, umfassend Behälter (34, 36, 38) für Kulturmedium, Wachstumsfaktoren und zu kultivierende Zellen, einen Inkubator (12) mit einer thermostatisierten Kammer (14), in der ein Behälter (54) zur Zellkultivierung oder Zellvermehrung untergebracht ist, sowie ein Steuerungscomputersystem (24) mit Mitteln zur Dateneingabe und -speicherung, das dazu bestimmt ist, die Kulturbedingungen im Innenraum zu regulieren und Ventile (20) zur Verteilung von Flüssigkeiten nach einer vordefinierten Abfolge zu steuern, **dadurch gekennzeichnet, dass** es eine Vorrichtung (16) zum Halten und Rühren des Zellkultur- oder Zellvermehrungsbehälters umfasst, die vom Computersystem gesteuert wird und in der Kammer untergebracht ist, und dadurch, dass der Behälter aus einem Zellvermehrungsbeutel (54) besteht, der mindestens eine mit den vorgenannten Behältern verbundene Einlassöffnung (68) und eine Auslassöffnung (64), die mit Mitteln (46) zur Rückgewinnung und Lagerung der Zellen nach der Kultivierung verbunden ist, wobei sich diese Lagermittel und die Reservoirs außerhalb des Gehäuses befinden und über Leitungen (66, 70, 72, 74) mit den Öffnungen des Zellvermehrungsbeutels verbunden sind, die zusammen mit dem Zellvermehrungsbeutel ein vor, das in die Kammer eingesetzt ist und die ein Wandelement der Kammer durchqueren, um die Zufuhr von Kulturmedium, Wachstumsfaktoren und zu kultivierenden Zellen in den Zellvermehrungsbeutel sowie die Rückgewinnung des Inhalts des Zellvermehrungsbeutels in die Lagereinrichtungen zu ermöglichen, wobei die Kammer geschlossen bleibt, wobei die Halte- und Rührvorrichtung (16) eine Halteplatte (110) für den Zellvermehrungsbeutel (54) umfasst, die um eine erste horizontale Achse (A) drehbar gelagert ist und um diese Achse zwischen einer im Wesentlichen horizontalen Position zur Zellkultivierung und einer im Wesentlichen vertikalen Position zur Entnahme der Zellen nach der Kultivierung bewegbar ist.

2. Automat gemäß Anspruch 1, **dadurch gekennzeichnet, dass** der Zellvermehrungsbeutel (54) ferner eine Entnahmeöffnung aufweist (58), der über eine Leitung (60) mit Entnahmevorrichtungen (62) verbunden ist, die sich außerhalb des Gehäuses (14) befinden, wobei diese Leitung den vorgenannten Teil des Inkubators durchquert und Teil des vormontierten Moduls ist.

3. Automat gemäß Anspruch 1 oder 2, **dadurch gekennzeichnet, dass** der Inkubator (12) einen Schrank umfasst, der eine Öffnung aufweist und mit einer Tür (50) zum dichten Verschließen dieser Öffnung ausgestattet ist, wobei die vorgenannten Leitungsdurchführungen (60, 66, 70) am Umfangsrand dieser Öffnung angebracht sind und im Wesentlichen parallele Nuten (82) zum Einführen der Leitungen aufweisen, wobei diese Nuten dazu bestimmt sind, von der Tür abgedeckt zu werden, wenn sich diese in geschlossener Position befindet.

4. Automat gemäß einem der vorstehenden Ansprüche, **dadurch gekennzeichnet, dass** die Behälter für Wachstumsfaktoren und zu kultivierende Zellen durch Taschen (36, 38) gebildet werden, die höher als die Einlassöffnung (68) der Zellvermehrungstasche (54) angeordnet sind, so dass der Inhalt jeder der Taschen für Wachstumsfaktoren und zu kultivierende Zellen durch Schwerkraft in die Zellvermehrungstasche fließen kann.

5. Automat gemäß einem der vorstehenden Ansprüche, **dadurch gekennzeichnet, dass** die Speichermittel eine oder zwei Taschen (46) umfassen, die zumindest teilweise tiefer liegen als die Auslassöffnung (64) der Zellausdehnungstasche (54), so dass nach der Kultivierung der Inhalt des Zellausdehnungsbeutels durch Schwerkraft in den oder die Beutel der Speichermittel fließen kann.

6. Automat gemäß einem der vorstehenden Ansprüche, **dadurch gekennzeichnet, dass** der Zellausdehnungsbeutel (54) flexible Wände aufweist, die flüssigkeitsdicht und gasdurchlässig, insbesondere für CO₂, sind und vorzugsweise Eigenschaften aufweisen, die die Anhaftung der zu kultivierenden Zellen an den Wänden des Beutels so weit wie möglich begrenzen.

7. Automat gemäß einem der vorstehenden Ansprüche, **dadurch gekennzeichnet, dass** er eine Peristaltikpumpe (22) zur Steuerung der Zufuhr des Kulturmediums in den Zellausdehnungsbeutel (54) umfasst und
Behälter (36, 38) für Wachstumsfaktoren und zu kultivierende Zellen, zum Spülen dieser Behälter.

8. Automat gemäß einem der vorstehenden Ansprüche, **dadurch gekennzeichnet, dass** er zwei Luftsammelkammern (40, 56) umfasst, von denen eine mit den Behältern (36, 38) für Wachstumsfaktoren und zu kultivierende Zellen verbunden ist und die andere mit der Zellausdehnungskammer (54) verbunden ist, und die dazu bestimmt sind, die in den Leitungen (70, 72, 74), der Zellausdehnungskammer (54) und/oder den Behältern (36, 38) enthaltene Luft aufzufangen und zu speichern.

9. Automat gemäß einem der vorstehenden Ansprüche, **dadurch gekennzeichnet, dass** die Leitungen durch flexible Schläuche (60, 66, 70, 72, 74, 76, 78) gebildet sind, von denen zumindest einige durch Ventile (86, 88, 90, 92, 94, 96, 98, 100, 102, 104, 106, 108) durchlaufen, die dazu bestimmt sind, diese Schläuche in der Schließstellung abzusperren.

10. Automat gemäß einem der Ansprüche 1 bis 9, **dadurch gekennzeichnet, dass** die Platte (110) um eine zweite horizontale Achse (B) drehbar gelagert ist, um die die Platte zum Rühren und Homogenisieren des Inhalts des Zellausdehnungsbeutels oszillieren soll.

11. Automat gemäß einem der Ansprüche 1 bis 10, **dadurch gekennzeichnet, dass** die Platte (110) Ventile (86, 88, 90) zur Steuerung der Zufuhr zum Zellausdehnungsbeutel (54), zur Entnahme des Inhalts dieses Beutels und zur Entnahme von Proben aus diesem Beutel trägt.

12. Automat gemäß einem der Ansprüche 1 bis 11, **dadurch gekennzeichnet, dass** die Halte- und Rührvorrichtung (16) einen vertikalen Arm (128) umfasst, der an seinem oberen Ende Mittel (130) zum Einhängen eines mit dem Zellausdehnungsbeutel (54) verbundenen Luftfangbeutels (56) aufweist.

13. Automat gemäß einem der vorstehenden Ansprüche, **dadurch gekennzeichnet, dass** die Lagereinrichtungen (46) um eine horizontale Achse drehbar gelagert sind und um diese Achse zwischen einer im Wesentlichen vertikalen Position und einer im Wesentlichen horizontalen Position bewegbar sind, wobei
wobei diese Mittel vollständig unterhalb des Zellausdehnungsbeutels angeordnet sind.

14. Steriles Einweg-Zellkulturset für einen Zellkulturautomaten (10), **dadurch gekennzeichnet, dass** es mindestens einen Kulturbeutel (54) und flexible Schläuche (60, 66, 70, 72, 74) zur Verbindung des Beutels mit anderen Beuteln oder Behältern umfasst, wobei die Schläuche und der Zellausdehnungsbeutel ein vormontiertes Modul bilden, wobei der Beutel dazu bestimmt ist, in eine Zellkulturkammer eingesetzt zu werden, und die Schläuche dazu bestimmt sind, ein Wandelement der genannten Kammer zu durchqueren, wobei der Zellkulturbag eine Einlassöffnung (68), eine Auslassöffnung (64) und gegebenenfalls eine Entnahmeöffnung (58) umfasst, wobei die Einlassöffnung (68) des Zellausdehnungsbeutels (54) über Schläuche (70, 72, 74) mit Einlass- und Auslassöffnungen eines Beutels (36) für Wachstumsfaktoren sowie mit Einlass- und Auslassöffnungen eines Beutels (38) für zu kultivierende Zellen verbunden ist, wobei das Kit ferner zwei Luftfallenbeutel (40, 56) umfasst, von denen einer mit den Auslassöffnungen der Beutel (36, 38) für Wachstumsfaktoren und zu kultivierende Zellen verbunden ist und der andere mit der Einlassöffnung (68) des Zellvermehrungsbeutels (54) verbunden ist.

15. Kit nach Anspruch 14, **dadurch gekennzeichnet, dass** es einen oder zwei Beutel (46) zur Rückgewinnung der Zellen nach der Kultur umfasst, die
über Schläuche (66) mit der Auslassöffnung (64) des Zellvermehrungsbeutels (54) verbunden sind.

16. Automat gemäß einem der Ansprüche 1 bis 13, umfassend eine Halte- und Rührvorrichtung (16) für einen Zellkulturautomaten (10), **dadurch gekennzeichnet, dass** er eine Halteplatte (110) für einen Zellvermehrungsbeutel (54) umfasst, wobei diese Platte drei Ventile (86, 88, 90) trägt und drehbar um eine erste horizontale Achse (A) zum Kippen der Platte aus einer im Wesentlichen horizontalen Position in eine im Wesentlichen vertikale Position sowie um eine zweite horizontale Achse (B) gelagert ist, um die die Platte zum Rühren oszillieren soll
und die Homogenisierung des Inhalts des Zellausdehnungsbehälters, wobei die Vorrichtung außerdem gesteuerte Mittel (124, 126) zum Kippen der Platte um die vorgenannten horizontalen Achsen umfasst.

17. Automat gemäß Anspruch 16, **dadurch gekennzeichnet, dass** er einen vertikalen Arm (128) umfasst, der an seinem oberen Ende Mittel (130) zum Einhängen eines Beutels (56) aufweist, der eine Luftfalle bildet.

18. Automatisiertes Verfahren zur Zellkultur mittels einer Vorrichtung (10) gemäß einem der Ansprüche 1 bis 13, 16 und 17, **dadurch gekennzeichnet, dass** es die folgenden Schritte umfasst:
a) Zuführen von Kulturmedium, Wachstumsfaktoren und anschließend der zu kultivierenden Zellen in den Zellvermehrungsbeutel (54), wobei die Kammer (14) des Inkubators (12) geschlossen gehalten wird;
b) Schütteln des Zellvermehrungsbeutels zur Homogenisierung seines Inhalts;
c) Halten des Zellvermehrungsbeutels unter Inkubationsbedingungen über einen Zeitraum von mehreren Tagen; und
d) den Inhalt des Zellvermehrungsbeutels in die Lagerungsvorrichtung (46) zu überführen, wobei der Innenraum geschlossen bleibt.

19. Verfahren nach Anspruch 18, **dadurch gekennzeichnet, dass** es umfasst:
- vor Schritt a) einen Schritt zum Einbau des vormontierten Moduls, indem der Zellausdehnungsbeutel (54) an der Rührvorrichtung (16) befestigt wird, die Leitungen (60, 66, 70, 72, 74) in die Durchflussvorrichtungen (80) des Inkubators (12) und in die Ventile (20) montiert werden, sowie durch Verbinden dieser Leitungen mit den Behältern oder Beuteln (34, 36, 38), und/oder
- vor Schritt a) einen Schritt zum Ablassen der in den Leitungen (70, 72, 74) enthaltenen Luft durch Durchleiten von Kulturmedium aus dem Kulturmediumbehälter (34) in den oder die Luftfallen bildenden Beutel (40, 56); und/oder
- nach der Zufuhr von Wachstumsfaktoren in den Zellvermehrungsbeutel in Schritt a) einen Schritt zum Spülen des Wachstumsfaktor-Behälters (36), indem Kulturmedium durch diesen Behälter geleitet und anschließend dessen Inhalt in den Zellvermehrungsbeutel (54) abgelassen wird; und/oder
- nach der Zufuhr von zu kultivierenden Zellen in den Zellvermehrungsbeutel in Schritt a) einen Schritt zum Spülen des Behälters (38) für zu kultivierende Zellen, indem Kulturmedium durch diesen Behälter geleitet und anschließend dessen Inhalt in den Zellvermehrungsbeutel (54) abgelassen wird; und/oder
- während des Schritts c) einen oder mehrere Schritte zur Entnahme von Proben aus dem Inhalt des Zellvermehrungsbeutels (54), denen jeweils ein Schritt zum Kippen der Trägerplatte (110) aus einer horizontalen Kulturposition in eine geneigte Position vorausgeht, in der die Entnahmeöffnung (58) des Beutels den tiefsten Punkt des Beutels darstellt; und/oder
- vor Schritt c) einen Schritt zum Entfernen der Behälter (34, 36, 38) für Kulturmedium, Wachstumsfaktoren und zu kultivierende Zellen, indem die Verbindungsleitung oder der Verbindungsschlauch (70) dieser Behälter zur Einlassöffnung (68) des Zellausdehnungsbeutels (54) durchtrennt und verschweißt oder abgeklemmt wird; und/oder
- vor oder während des Schritts d) das Kippen der Platte (110) in eine im Wesentlichen vertikale Position, so dass die Auslassöffnung (64) des Zellausdehnungsbeutels (54) den tiefsten Punkt des Beutels darstellt.

20. Verwendung einer Vorrichtung gemäß einem der Ansprüche 1 bis 13, 16, 17 oder eines Kits gemäß Anspruch 14 oder 15 zur Kultivierung von CD34+-Stammzellen oder mononukleären Blutzellen, wie beispielsweise Lymphozyten.

21. Verwendung nach Anspruch 20, **dadurch gekennzeichnet, dass** die Stammzellen aus einer oder mehreren Quellen stammen, wie beispielsweise
insbesondere Nabelschnurblut, Knochenmark und Vollblut.

## Claims

1. Automated (10) apparatus of cell culture, comprising tanks (34, 36, 38) of culture medium, growth factors and cells to be cultured, an incubator (12) with a thermostated enclosure (14) in which is housed a container (54) for cell culture or expansion, and a control computer system (24) including means configured for entering and recording data intended to control the culture conditions in the enclosure and to manage valves (20) for dispensing fluids in a predefined sequence, **characterized in that** it comprises an agitation device (16) for supporting and agitating the cell culture or expansion container which is controlled by the control computer system and which is disposed in said enclosure, and **in that** said container is formed by a cell expansion bag (54) having at least one inlet port (68) connected to said tanks and one outlet port (64) connected to means (46) configured for harvesting and storage of the cells after culture, these harvesting and storage means and said tanks being located outside the enclosure and being connected to said ports of said cell expansion bag by conduits (66, 70, 72, 74) which form with said cell expansion bag a preassembled module placed in said enclosure and which pass through a wall of said enclosure so as to allow to feed the cell expansion bag with said culture medium, growth factors and cells to be cultured, and to harvest the contents of said cell expansion bag in the harvesting and storage means while maintaining the enclosure closed, the agitation device (16) comprising a tray (110) for supporting the cell expansion bag (54), the tray being rotatably mounted about a first horizontal axis (A), the tray being moveable about this axis between a substantially horizontal position of cell culture to a substantially vertical position of harvesting cells after culture.

2. Automated apparatus according to claim 1, wherein said cell expansion bag (54) further comprises a sampling outlet (58) which is connected by a conduit (60) to a sampling device (62) located outside of said enclosure (14), said conduit passing through said wall of said incubator and being part of said preassembled module.

3. Automated apparatus according to claim 1 or 2, wherein said incubator (12) includes a cabinet having an opening and equipped with a sealed closure door (50), means (80) for the passageway of said conduits (60, 66, 70) being mounted on the peripheral edge of said opening and having grooves (82) which are slightly parallel and into which are engaged said conduits, said grooves being intended to be covered by said sealed closure door when in closed position.

4. Automated apparatus according to one of the preceding claims, wherein said tanks of growth factors and of cells to be cultured are formed by bags (36, 38) which are located above the inlet port (68) of said cell expansion bag (54), so that the content of each of the bags of growth factors and of cells to be cultured can flow by gravity to said cell expansion bag.

5. Automated apparatus according to one of the preceding claims, wherein said harvesting and storage means comprises one or two bags (46) which are at least partially located below the outlet port (64) of said cell expansion bag (54) so that, after culturing, the content of said cell expansion bag can flow by gravity to said one or two bags of the harvesting and storage means.

6. Automated apparatus according to one of the preceding claims, wherein said cell expansion bag (54) comprises flexible liquid-tight and gas permeable walls, in particular to CO₂, and preferably having properties that minimize the adhesion of the cells to be cultured to the walls of the bag.

7. Automated apparatus according to one of the preceding claims, which comprises a peristaltic pump (22) for controlling the supply of said culture medium to said cell expansion bag (54) and to said tanks (36, 38) of growth factors and of cells to be cultured, for rinsing said tanks.

8. Automated apparatus according to one of the preceding claims, which comprises two bags (40, 56) forming air trap, one of which being connected to the tanks (36, 38) of growth factors and of cells to be cultured, and the other being connected to said cell expansion bag (54), said two bags being intended to collect and store the air contained in said conduits (70, 72, 74), said cell expansion bag (54) and/or said tanks (36, 38).

9. Automated apparatus according to one of the preceding claims, wherein said conduits are formed by flexible tubes (60, 66, 70, 72, 74, 76, 78), some of which going through valves (86, 88, 90, 92, 94, 96, 98, 100, 102, 104, 106, 108) which are intended in closed position, to pinch the tubes.

10. Automated apparatus according to one of the claims 1 to 9, wherein said tray (110) is mounted in rotation around a second horizontal axis (B) around which the tray is intended to oscillate for agitation and homogenization of the content of said cell expansion bag.

11. Automated apparatus according to one of the claims 1 to 10, wherein the tray (110) bears valves (86, 88, 90) for controlling the supply of said cell expansion bag (54), the harvesting of the content of said cell expansion bag, and the sampling of said cell expansion bag.

12. Automated apparatus according to one of the claims 1 to 11, wherein the supporting and agitation device (16) comprises a vertical arm (128) having at its upper end means (130) for attaching a bag (56) forming an air trap connected to said cell expansion bag (54).

13. Automated apparatus according to one of the preceding claims, wherein said means for harvesting and storage (46) are mounted in rotation around a horizontal axis and are movable around said axis between a substantially vertical position and a substantially horizontal position in which said means for harvesting and storage are located completely below said cell expansion bag.

14. Sterile and disposable cell culture kit for a cell culture automated apparatus (10), which comprises at least one cell expansion bag (54) and flexible tubes (60, 66, 70, 72, 74) connecting said at least one cell expansion bag to other bags or tanks, said tubes and said cell expansion bag being pre-assembled, the bag being intended to be placed within a cell culture enclosure and the tubes being intended to pass through a wall element of said enclosure, the cell expansion bag comprising an inlet port (68), an outlet port (64), and optionally a sampling port (58), the inlet port (68) of the cell expansion bag (54) being connected by tubes (70, 72, 74) to the inlet and outlet ports of a growth factor bag (36), and to the inlet and outlet ports of a bag (38) for cell culture, the kit further comprising two bags (40, 56) forming air trap, one of which is connected to the outlet ports of the growth factor and cell culture bags (36, 38), and the other of which is connected to the inlet port (68) of the cell expansion bag (54).

15. Kit according to claim 14, wherein it comprises one or two bags (46) for harvesting the cells after culture, which are connected by tubes (66) to said outlet port (64) of said cell expansion bag (54).

16. Automated apparatus according to one of the claims 1 to 13, comprising a supporting and agitation device (16) for a cell culture automated apparatus (10), **characterized in that** it comprises a tray (110) for supporting a cell expansion bag (54), this tray bearing three valves (86, 88, 90) and being mounted in rotation about a first horizontal axis (A) for the tilting of said tray from a substantially horizontal position to a substantially vertical position, and about a second horizontal axis (B) around which the tray is intended to oscillate for agitation and homogenization of the contents of said cell expansion bag, wherein the device further comprises controlled means (124, 126) for tilting the tray around the aforesaid horizontal axes.

17. Automated apparatus according to claim 16, which comprises a vertical arm (128) having at its upper end means (130) for attaching a bag (56) forming an air trap.

18. An automated method of cell culture by means of an automated apparatus (10) according to one of claims 1 to 13, 16 and 17, which comprises the steps of:
a) feeding said cell expansion bag (54) with said culture medium, with said growth factors then with said cells to be cultured, while maintaining said enclosure (14) of said incubator (12) closed;
b) agitating said cell expansion bag in order to homogenize its content;
c) maintaining said cell expansion bag in incubation conditions for a period of several days, and
d) harvesting the content of said cell expansion bag in said harvesting and storage means (46) while maintaining the enclosure closed.

19. Method of claim 18, which comprises:
- prior to step a), a step of installing said preassembled module by fitting said cell expansion bag (54) on said supporting and agitation device (16), by mounting said conduits (60, 66, 70, 72, 74) in the passageway means (80) of said incubator (12) and into said valves (20), and by connecting said conduits to tanks or bags (34, 36, 38), and/or
- prior to step a), a step of evacuating air contained in said conduits (70, 72, 74) by passage of culture medium from said culture medium tank (34) to said bags (40, 56) forming air trap, and/or
- after supplying said cell expansion bag with growth factors in step a), a step of rinsing said growth factors tank (36) by flowing culture medium in said growth factors tank and then by draining its content to said cell expansion bag (54), and/or
- after supplying said cell expansion bag with cells to be cultured in step a), a step of rinsing the tank (38) containing said cells to be cultured by flowing culture medium in said tank of cells to be cultured and then by draining its content to said cell expansion bag (54), and/or
- during step c), one or more steps of sampling the contents of said cell expansion bag (54), which are each preceded by a step of tilting said tray from a horizontal position of cultivation to an inclined position in which the sampling outlet (58) of said cell expansion bag is the lowest point of this bag; and/or
- prior to step c), a step of removing the tanks (34, 36, 38) of culture medium, of growth factors and of cells to be cultured by cutting and welding or pinching the conduit or tube (70) connecting these tanks to the inlet port (68) of said cell expansion bag (54), and/or
- before or during step d), a step of tilting said tray (110) in a substantially vertical position so that the outlet port (64) of said cell expansion bag (54) represents the lowest point of this bag.

20. Use of an automated apparatus according to claim 1 to 13, 16, 17 or of a cell culture kit according to claims 14 or 15, for the culture of stem cells of type CD34 + or blood mononuclear cells, such as for example lymphocytes.

21. Use according to claim 20, herein the stem cells are issued from one or more sources, more particularly such as umbilical cord blood, bone marrow and and/or whole blood.
